Europäisches Patentamt

European Patent Office

Office européen des brevets

(19)

(11) EP 0 839 204 B1

(12) **FASCICULE DE BREVET EUROPEEN**

(45) Date de publication et mention
de la délivrance du brevet:
**14.09.2005 Bulletin 2005/37**

(51) Int Cl.7: **C12N 15/82**, C12N 15/12,
C07K 14/805, C12N 5/10,
A01H 5/00, A61K 38/42

(21) Numéro de dépôt: **96925810.2**

(22) Date de dépôt: **17.07.1996**

(86) Numéro de dépôt international:
**PCT/FR1996/001123**

(87) Numéro de publication internationale:
**WO 1997/004115 (06.02.1997 Gazette 1997/07)**

(54) **PROCEDE DE PRODUCTION, PAR DES CELLULES VEGETALES, DE PROTEINES HEMINIQUES, PROTEINES AINSI OBTENUES ET PRODUITS CONTENANT CES PROTEINES**

VERFAHREN ZUR HERSTELLUNG VON HÄM-ENTHALTENDE PROTEINE DURCH PFLANZENZELLEN, HERGESTELLTE PROTEINE UND DIESE ENTHALTENDE PRODUKTEN

METHOD FOR PRODUCING HAEMIN PROTEINS USING PLANT CELLS, RESULTING PROTEINS AND PRODUCTS CONTAINING SAME

(84) Etats contractants désignés:
**AT BE CH DE DK ES FI FR GB GR IE IT LI LU MC NL PT SE**

(30) Priorité: **17.07.1995 FR 9508615**

(43) Date de publication de la demande:
**06.05.1998 Bulletin 1998/19**

(73) Titulaires:
• **MERISTEM THERAPEUTICS**
**63100 Clermont-Ferrand (FR)**
• **INSTITUT NATIONAL DE LA SANTE ET DE LA RECHERCHE MEDICALE (INSERM)**
**75654 Paris Cédex 13 (FR)**

(72) Inventeurs:
• **MEROT, Bertrand**
**F-63530 Volvic (FR)**
• **DIERYCK, Wilfrid**
**F-77178 Saint-Pathus (FR)**
• **LENEE, Philippe**
**F-98800 Nouméa (FR)**
• **MARDEN, Michael**
**F-93600 Aulnay-sous-Bois (FR)**
• **GRUBER, Véronique**
**F-63400 Chamalières (FR)**
• **PAGNIER, Renée-Josée**
**F-94270 Le Kremlin-Bicêtre (FR)**
• **BAUDINO, Sylvie**
**F-63870 Orcines (FR)**

• **POYART, Claude**
**F-75013 Paris (FR)**

(74) Mandataire: **Thurgood, Alexander John**
**Cabinet Richebourg**
**"Le Clos du Golf"**
**69, rue Saint-Simon**
**42000 Saint-Etienne (FR)**

(56) Documents cités:
EP-A- 0 273 889            WO-A-91/06320
WO-A-93/03161             WO-A-93/08831
WO-A-93/25697

• TRANSFUSION CLINIQUE ET BIOLOGIQUE, vol. 2, no. 6, 1995, pages 441-447, XP000614371 DIERYCK, W., ET AL.: "Recombinant protein expression in plants"
• PROCEEDINGS OF THE NATIONAL ACADEMY OF SCIENCES OF USA, vol. 90, WASHINGTON US, pages 11811-11815, XP002023062 OHME-TAKAGI, M., ET AL.: "The effect of sequences with high AU content on mRNA stability in tobacco"
• BIOTECHNOLOGY, vol. 9, no. 1, pages 57-61, XP002023063 WAGENBACH, M., ET AL.: "Synthesis of wild type and mutant human hemoglobins in Saccharomyces cerevisiae"

• **PROCEEDINGS OF THE NATIONAL ACADEMY OF SCIENCES OF USA, vol. 88, no. 16, 15 Août 1991, pages 7041-7045, XP000272765 KAZUKI SAITO ET AL: "INTEGRATION AND EXPRESSION OF A RABBIT LIVER CYTOCHROME P-450 GENE IN TRANSGENIC NICOTIANA TABACUM"**

**Description**

L'invention concerne un procédé de production, par des cellules végétales, de protéines héminiques, et en particulier les protéines héminiques capables de fixer réversiblement l'oxygène, par exemple l'hémoglobine et ses dérivés, et la myoglobine. Elle concerne en outre les protéines obtenues par la mise en oeuvre du procédé. L'invention vise également les cellules et plantes génétiquement transformées capable de produire ces protéines, et les construits d'acides nucléiques impliqués dans la transformation. En outre, l'invention vise des produits par exemple, pharmaceutiques ou cosmétiques, contenant ces protéines héminiques.

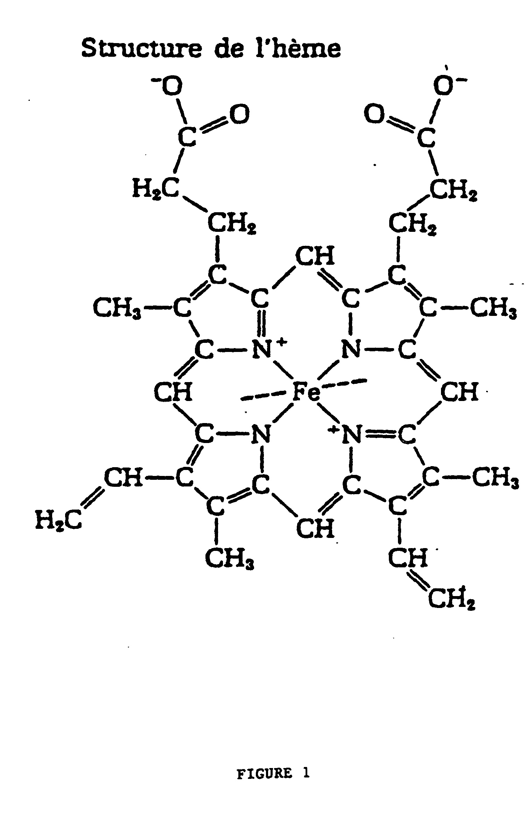

Les protéines héminiques sont des molécules complexes composées d'une ou plusieurs chaîne(s) polypeptidique(s) en association à un ou plusieurs noyau(x) ferroporphyrique(s). Ces noyaux sont composés de quatre cycles pyrrole, juxtaposés en une structure fermée et reliés par des ponts méthéniques, et contenant au centre de la molécule, un atome de fer. Les protéines héminiques se distinguent les unes des autres par la nature et le nombre des chaînes polypeptidiques et par la nature des chaînes latérales portées par les huit atomes β des cycles pyrrole. Un exemple de noyau ferroporphyrique est la protoporphyrine IX à fer également connue sous le nom de "protohème" ou simplement "hème" (figure 1).

La famille des protéines héminiques comprend de nombreuses substances importantes sur le plan biologique chez les animaux et chez les plantes, notamment l'hémoglobine, la myoglobine, les cytochromes, les peroxydases, les catalases.

L'hémoglobine est le constituant principal des globules rouges du sang. Elle a pour fonction essentielle de fixer, transporter et délivrer la quantité d'oxygène nécessaire au fonctionnement normal des tissus.

La molécule d'hémoglobine est composée de deux types de chaînes ou sous-unités de globine, nommées $\alpha$ et $\beta$ (de 141 et 146 acides aminés respectivement), et appariées deux à deux pour former un tétramère $\alpha_2\beta_2$. Chacune de ces sous-unités renferme, solidement attachée dans une poche hydrophobe, une molécule d'hème (c'est-à-dire la protoporphyrine IX) contenant en son centre un atome de fer divalent ($Fe^{2+}$) auquel se lie de façon réversible une molécule d'oxygène. Chaque molécule d'hémoglobine tétramérique contient donc 4 atomes de fer et 4 molécules d'oxygène qu'elle fixe lors de son passage dans les poumons. La masse moléculaire du tétramère est de 64 650 D. Chez l'homme, les chaînes $\alpha$ et $\beta$ sont synthétisées à partir de deux types de gènes situés sur les chromosomes 16 et 11 respectivement.

Le terme de chaînes de type bêta, ou "non alpha", recouvre, non seulement les chaînes bêta, mais également les chaînes dénommées epsilon, gamma ou delta.

Normalement, chez l'adulte, plus de 95% de l'hémoglobine est constituée par du tétramère $alpha_2$ bâta$_2$, c'est-à-dire l'association de deux dimères hétérologues alpha-bêta, associés au complexe catalytique, l'hème. On trouve 2% à 3% d'une hémoglobine constituée de tétramères $alpha_2$ delta$_2$, et des traces d'hémoglobine foetale $alpha_2$ gamma$_2$.

La molécule d'hémoglobine humaine tétramérique existe sous deux formes ou structures quaternaires selon que l'oxygène est lié ou non aux atomes de fer. En présence d'oxygène, l'hémoglobine est dite en état R (pour relâchée) et son affinité pour l'oxygène est forte. En l'absence d'oxygène, l'hémoglobine est dite en état T (pour tendue) et son affinité pour l'oxygène est 100 fois plus faible (Perutz, 1970). L'affinité résultante est liée à l'équilibre entre les concentrations de formes R et T. Cette affinité est d'autant plus faible que la concentration de l'hémoglobine sous forme T est plus élevée à tout niveau d'oxygénation. L'affinité de l'hémoglobine pour l'oxygène est réglée par le co-facteur - 2,3-diphosphoglycérate (DPG), une petite molécule provenant du métabolisme du glucose et qui se fixe sur les chaînes β de l'hémoglobine tétramérique diminuant son affinité pour l'oxygène.

L'augmentation des risques infectieux par des produits dérivés du sang humain (hépatites, H.I.V) rend nécessaire la mise au point d'un transporteur artificiel d'oxygène comme substitut à la transfusion sanguine.

Des techniques mettant en oeuvre les ADN recombinants ont été proposées pour produire les chaînes protéiques de la globine.

Les premières techniques mises en oeuvre avaient essentiellement pour but de faire synthétiser dans E. coli, les chaînes alpha et bêta séparément (Nagaï et Thogen-Sen, 1987), impliquant des procédés lourds d'expression séparée de chacune des chaînes. Ces procédés pouvaient difficilement être exploités à l'échelle industrielle.

Plus récemment, l'expression d'hémoglobine recombinante soluble et fonctionnelle a été mise au point dans E. coli (Hoffman et al., 1990, P.N.A.S., 87, 8521-8525) et Saccharomyces cerevisiae (Wagenbach et al., 1991, Biotechnology, 9, 57-61). Chacun de ces systèmes présente avantages et inconvénients. En effet, les taux d'expression les plus élevés sont obtenus dans E. coli qui présente néanmoins le désavantage de produire des endotoxines et de ne pas cliver les méthionines $NH_2$-terminales contrairement à Saccharomyces cerevisiae. Dans la levure, les rendements de synthèse d'hémoglobine sont faibles (3 à 5%), comparés aux 10-15% obtenus dans E. coli. Ceci limite actuellement l'utilisation de la levure dans le cadre d'un projet de développement industriel.

L'utilisation de cellules animales en culture ou d'animaux transgéniques comme hôtes de production a également été réalisée (Swanson et al., Bio/Technology, Mai 1992, 10, page 55). Ces techniques ne paraissent pas exploitables actuellement en raison des faibles taux d'expression et les risques de contaminations virales et par des prions.

Le problème technique que se propose de résoudre la présente invention est de produire des protéines héminiques, et notamment l'hémoglobine et ses dérivés, en grande quantité à des coûts faibles, sans risque de contaminations virales ou sub-virales. Les inventeurs ont apporté une solution à ce problème en utilisant comme hôte pour la transformation et la production, des cellules végétales.

Diverses équipes se sont déjà intéressées à la production de protéines recombinantes de mammifères dans les cellules végétales ou dans des plantes transgéniques. Par exemple, l'expression spécifique dans les graines de colza, de la leu-enképhaline a été obtenue avec des niveaux d'expression d'environ 0,1% (Vanderkerckhove et al., Biotechnology, 1989, 7, 929-932).

En 1990, Sijmons et al., (Biotechnology, 1990, 8, 217-221) ont transféré le gène de la sérum albumine humaine dans des cellules de tabac et de pomme de terre. Quelle que soit l'origine des peptides signaux (humaine ou végétale), des taux de sérum albumine humaine de l'ordre 0,02% des protéines totales ont été obtenus dans les feuilles, les tiges et les tubercules de pomme de terre.

D'autres protéines recombinantes de mammifères ont été aussi produites dans les plantes : l'antigène de surface de l'hépatite B (Mason et al., P.N.A.S., 1992, 89, 11745-11749) ; l'interféron humain (Edelbaum J. of Interferon Res., 1992, 12, 449-453) ; un anticorps de souris anti-Streptococcus mutans, agent de la carie dentaire (Hiatt et Ma, FEBS, 1992, 307, 71-75) ; un anticorps anti-Herpès (Russel, 1994) et l'hirudine (Moloney, 1994).

L'ensemble de ces recherches montre que la production de protéines recombinantes de mammifères dans les cellules végétales est possible et que les mécanismes de synthèse de protéines à partir des séquences d'ADN sont similaires chez les cellules animales et les cellules végétales.

En revanche, il existe peu d'informations au sujet des porphyrines à fer chez les végétaux, notamment sur leurs structures, leurs voies de synthèse et l'assemblage des noyaux porphyriques et les chaînes protéiques pour former les protéines héminiques. La production de molécules recombinantes ayant la capacité de fixer réversiblement l'oxygène, et nécessitant l'assemblage, dans la cellule, de protéines hétérologues et de porphyrines endogènes végétales n'a jamais été décrite.

L'invention concerne un procédé de production, par des cellules végétales, de protéines héminiques recombinantes. Selon le procédé de l'invention, la cellule végétale est modifiée génétiquement afin de pouvoir exprimer le composant protéique d'une protéine héminique. Le noyau porphyrique est produit par la cellule de manière endogène, l'assemblage des composants protéiques et porphyriques ayant lieu spontanément grâce à leur affinité élevée l'un pour l'autre.

Plus particulièrement, l'invention concerne un procédé de production de protéines héminiques comprenant les étapes suivantes :

i) introduction, dans des cellules végétales, d'une ou plusieurs molécule(s) d'acide nucléique dont chacune comporte au moins une séquence codant un composant protéique d'une protéine héminique d'origine animale ou pour une variante ou une partie de ce composant protéique, et éventuellement une séquence codant un agent de sélection ;

ii) sélection des cellules ayant intégré l'acide nucléique codant pour le composant protéique ;

iii) propagation des cellules transformées, soit en culture, soit par la régénération de plantes entières transgéniques ou chimériques ;

iv) récupération, et éventuellement purification, d'une protéine héminique comprenant un assemblage de la protéine ou des protéines codées par le susdit acide nucléique avec au moins un noyau porphyrique à fer, ou une pluralité de ces assemblages.

L'invention concerne de préférence un procédé de production de protéines héminiques comprenant les étapes suivantes :

i) introduction, dans des cellules végétales, d'une ou plusieurs molécule(s) d'acides nucléiques dont chacune comporte au moins une séquence codant pour un composant protéique d'une protéine héminique d'origine animale de préférence capable de fixer réversiblement l'oxygène, ou pour une variante ou une partie de ce composant protéique, et éventuellement une séquence codant pour un agent de sélection ;

ii) sélection des cellules contenant l'acide nucléique codant pour le composant protéique de la protéine héminique ;

iii) éventuellement, propagation des cellules transformées, soit en culture, soit par la régénération de plantes entières transgéniques ou chimériques ;

iv) récupération, et éventuellement purification, d'une protéine héminique comprenant un complexe constitué de la protéine ou des protéines codées par le susdit acide nucléique et au moins un noyau porphyrique à fer, ou une pluralité de ces complexes.

Dans le contexte de la présente invention, le terme "protéine héminique" signifie toute protéine ayant un noyau porphyrique à fer comme groupement prosthétique, et en particulier la protoporphyrine IX telle qu'elle existe dans l'hémoglobine et la myoglobine humaine (figure 1). Le noyau porphyrique peut également être des dérivés de l'hème de celles de l'hème humaine. De préférence, les chaînes latérales sont hydrophobes.

Les protéines héminiques de l'invention englobent en particulier les protéines héminiques ayant comme fonction principale la fixation réversible de l'oxygène, c'est-à-dire la myoglobine et l'hémoglobine, ainsi que les cytochromes dont le rôle est de transporter des électrons. Les dérivés de ces protéines conservant ces fonctionnalités sont aussi englobés par l'invention.

Selon une variante préférée, la protéine héminique de l'invention est l'hémoglobine ou une protéine de type hémoglobine. Dans le contexte de l'invention, le terme "protéine de type hémoglobine" englobe toutes les protéines héminiques présentant à la fois :

   i) une ou plusieurs chaîne(s) d'α- et/ou de β-globine, ou de variantes de ces polypeptides, et
   ii) une ou plusieurs molécules de protoporphyrine IX à fer, ou de protoporphyrines se distinguant de la protoporphyrine IX par la nature, des chaînes latérales,
   iii) ayant une capacité de fixer réversiblement l'oxygène, de préférence avec une affinité comprise entre 10 et 50 mm Hg à 37°C, pH7.4. Plus particulièrement, l'affinité est comprise entre 20 et 30 mm Hg, à titre d'exemple, la $P_{50}$ du sang total à pH 7.2 et de $26 \pm 2$ mm Hg.

Dans ce qui suit, le terme "molécule de type hémoglobine" sera utilisé de manière synonyme avec le terme "dérivé de l'hémoglobine".

Dans ce contexte, une "variante" d'un composant protéique, et particulièrement de l'α- ou β-globine, signifie une séquence en acides aminés qui se différencie par rapport à la séquence naturelle, par une ou plusieurs substitution(s), délétion(s) ou insertion(s) d'acides aminés. En général, la variante présente au moins 90%, et de préférence au moins 95% d'homologie, ou d'identité avec la séquence naturelle. Dans le contexte de la présente invention, le pourcentage d'homologie entre deux séquences d'acides aminés est calculé comme étant le nombre d'acides aminés identiques plus le nombre d'acides aminés similaires dans l'alignement des deux séquences, divisé par la longueur des séquences entre deux positions données. Si, entre les deux positions données, les deux séquences n'ont pas la même longueur, le pourcentage d'homologie est le nombre d'acides aminés identiques et similaires, divisé par la longueur de la séquence la plus longue. Les acides aminés considérés comme étant "similaires" sont connus dans l'art, voir par exemple R.F. Feng, M.S. Jobson and R.F. Doolittle ; J. Mol. Evol. ; 1985 ; 21 ; 112-115. Ils sont normalement considérés comme étant ceux qui, au sein d'une matrice de permutation, ont un coefficient de substitution positif.

Le terme "variante" englobe aussi des fragments de chaînes polypeptidiques, par exemple de l'α- ou de la β-globine, ayant normalement une longueur d'au moins 90% de la molécule mère. Les variantes peuvent également être rendues plus longues que la molécule mère par l'addition de séquences non-fonctionnelles. De préférence, les variantes conservent les propriétés biologiques et immunologiques de la molécule mère.

La première étape du procédé de l'invention consiste en l'introduction, dans des cellules végétales, d'une ou plusieurs molécule(s) d'acide nucléique comportant au moins une séquence codant un composant protéique d'une protéine héminique de mammifère, ou pour une variante de ce composant.

Lorsque la protéine héminique est une protéine à chaîne unique, par exemple la myoglobine ou le cytochrome, l'acide nucléique introduit dans les cellules végétales comporte normalement une copie de la séquence codant pour cette protéine.

En revanche, lorsqu'il s'agit d'une protéine oligomérique ou multimérique, telle que l'hémoglobine ou des molécules du type hémoglobine, les séquences codant les différentes unités protéiques sont introduites dans la cellule végétale, soit au sein de la même molécule d'acide nucléique, soit au sein de molécules d'acides nucléiques distinctes. De préférence, pour la production d'hémoglobine et ses dérivés, les séquences codant l'α- et β-globine, ou leurs variantes, se trouvent au sein du même vecteur, dit vecteur de co-expression. Le vecteur peut comporter une ou plusieurs copie(s) de chaque séquence codante.

Alternativement, les séquences codant l'α- et la β-globine, ou leurs variantes, peuvent se trouver sur des molécules d'acides nucléiques distinctes. Selon cette variante, les deux molécules peuvent être introduites dans la même cellule végétale, à condition de disposer d'un système de sélection approprié. Une autre technique consiste en l'introduction de l'une des molécules dans une première cellule végétale, et l'autre dans une deuxième cellule végétale. Chacune des cellules transformées est ensuite régénérée en plante entière, les plantes ainsi obtenues pouvant alors être croisées pour donner lieu à une descendance capable de produire à la fois les chaînes α et β. Cette approche peut être utilisée pour optimiser le rendement de l'hémoglobine.

Les molécules d'acide nucléique introduites dans la cellule végétale lors de la première étape du procédé font également partie de l'invention. De manière générale, ces acides nucléiques comportent :

i) une ou plusieurs séquence(s) codant un composant protéique d'une protéine héminique animale, et

ii) une ou plusieurs séquence(s) codant un signal d'adressage d'origine végétale, et/ou des séquences régulatrices de transcription reconnues par une cellule végétale.

Plus particulièrement, l'acide nucléique de l'invention comporte :

i) une ou plusieurs séquence(s) codant un composant protéique d'une protéine héminique animale, ladite protéine ayant la capacité de fixer réversiblement l'oxygène, et

ii) des séquences régulatrices de transcription reconnues par une cellule végétale, comprenant un promoteur et des séquences régulatrices de terminaison, et

iii) une ou plusieurs séquence(s) codant un signal d'adressage d'origine végétale.

De préférence, les séquences codant le composant protéique codent l'α- ou la β-globine animale, par exemple humaine ou bovine, ou les variantes de celles-ci. De cette façon, les propriétés de la molécule, et notamment l'affinité pour l'oxygène et la stabilité, peuvent être optimisées.

Parmi ces modifications, il est possible par exemple d'introduire dans l'une ou dans les deux chaînes α- et β-globine, par mutagénèse dirigée, une ou deux différence(s) de séquences pour en diminuer l'affinité pour l'oxygène. Ces mutations peuvent être choisies à partir d'exemples de mutations naturelles (voir tableau I), soit des mutations indiquées par l'examen du modèle tridimensionnel de l'hémoglobine A naturelle.

Tableau I :

| Quelques hémoglobines humaines mutées (Int. Hemoglobin Center, 1995) | | |
|---|---|---|
| Hémoglobine anormale | Positions et résidus normaux | Remplacement |
| | chaîne α | |
| I | 16 Lys | Glu |
| $G_{Honolulu}$ | 30 Glu | Gln |
| Norfolk | 57 Gly | Asp |
| $M_{Boston}$ | 58 His | Tyr |
| $G_{Philadelphia}$ | 68 Asn | Lys |
| $O_{Indonesia}$ | 116 Glu | Lys |
| | chaîne β | |
| C | 6 Glu | Lys |
| S | 6 Glu | Val |
| $G_{San José}$ | 7 Glu | Gly |
| E | 26 Glu | Lys |
| $M_{Saskatoon}$ | 63 His | Tyr |
| Zürich | 63 His | Arg |
| $M_{Milwaukee}$ | 67 Val | Glu |
| $D_{Punjab}$ | 121 Glu | Gln |
| Mequon | 41 Phe | Tyr |
| Providence | 82 Lys | Asp |

De façon très avantageuse, on utilisera les mutants dont les propriétés fonctionnelles correspondent aux conditions physiologiques du transport de l'oxygène : liaison réversible, coopérativité et faible vitesse d'autooxydation. Parmi les mutants, on utilisera de préférence les doubles mutants $\alpha_2\beta_2$F41Y,K82D (c'est-à-dire un mutant dont la chaîne β comporte les modifications suivantes : Phe-41 est remplacé par Tyr, et Lys-82 est remplacé par Asp) ou $\alpha_2\beta_2$F41Y,K66T (c'est-à-dire un mutant dont la chaîne β comporte les modifications suivantes : Phe-41 est remplacé par Tyr, et Lys-66 est remplacé par Thr) qui correspondent à ces caractéristiques fonctionnelles.

La modification des chaînes α et β peut aussi être effectuée afin de stabiliser la molécule, c'est-à-dire d'éviter la dissociation du tétramère en dimères de petites tailles rapidement filtrés par les reins et limitant la durée de vie intra-vasculaire de l'hémoglobine. Le pontage covalent, à l'aide de phosphate ou de diaspirine, a été démontré comme étant une technique efficace pour stabiliser le tétramère (Benesch et Kwong, 1994). Le même résultat peut être obtenu par le biais de modifications de la chaîne en acides aminés. Les sous-unités α sont produites sous forme dimérique alpha-alpha liée par un résidu glycyl. Sous cette forme, elles conservent leur capacité de s'assembler correctement aux sous-unités partenaires bêta et à l'hème pour former une hémoglobine soluble. Cette hémoglobine ne peut plus se dissocier en dimères car la structure tétramérique est stabilisée par une liaison covalente (liaison peptidique) entre les dimères alpha-bêta. Cette technique permet d'augmenter la demie vie intra-vasculaire de la molécule.

Parmi les variantes, il est également possible d'utiliser une protéine hybride composée d'une partie de la chaîne alpha et une partie de la chaîne bêta.

Selon une variante préférée de l'invention, l'acide nucléique comprend, outre les séquences codant l'α- ou β-globine, des séquences codant des signaux d'adressage. De préférence, ces signaux sont des signaux d'adressage chloroplastiques ou mitochondriaux. L'expression et/ou l'accumulation des protéines recombinantes dans ces organites est particulièrement préférée en raison de la disponibilité de porphyrines à fer endogènes que l'on trouve ici. Le rendement en protéines héminiques est donc augmenté. En outre, l'adressage des protéines vers les chloroplastes et les mitochondries évite la glycosylation de la protéine, ce qui peut être avantageux dans la mesure où la molécule d'hémoglobine naturelle n'est pas glycosylée.

Comme exemple de signaux d'adressage chloroplastiques, on peut citer la séquence codant pour le peptide transit du précurseur de la petite sous-unité de la ribulose 1,5-biphosphate carboxylase de Pisum sativum (voir exemples). Comme signaux d'adressage mitochondrial, on peut citer la séquence codant pour le peptide transit du précurseur de la sous-unité bêta de l'ATP-aseF1 mitochondriale de Nicotiana plumbaginifolia (voir exemples).

Ces peptides transits, ainsi que la méthionine N-terminale, sont normalement clivés dans les chloroplastes ou les mitochondries. L'expression des protéines dans les plastes a donc également l'avantage de produire une molécule dépourvue de la méthionine N-terminale comme la molécule naturelle.

Selon une autre variante, les séquences d'adressage peuvent être des séquences codant un peptide signal N-terminal ("prépeptide"), éventuellement en association à un signal responsable de la rétention de la protéine dans le réticulum endoplasmique (signal du type KDEL), ou un signal d'adressage vacuolaire ou "propeptide". La présence du peptide signal N-terminal ou prépeptide permet la pénétration de la protéine naissante dans le réticulum endoplasmique où ont lieu un certain nombre de maturations post-traductionnelles, notamment le clivage du peptide signal, les N-glycosylations, si la protéine en question présente des sites de N-glycosylation, et la formation des ponts disulfures. Parmi ces différents signaux, le prépeptide, responsable de l'adressage de la protéine dans le réticulum endoplasmique, est dominant. Il s'agit normalement d'un peptide signal N-terminal hydrophobe ayant entre 10 et 40 acides aminés et étant d'origine animale ou végétale. De préférence, il s'agit d'un prépeptide d'origine végétale, par exemple celui de la sporamine, de la lectine d'orge, de l'extensine végétale, de l'α-mating factor, de la protéine de pathogénèse 1 ou 2.

Normalement, le peptide signal est clivé par un signal peptidase dès l'introduction co-traductionnelle du polypeptide naissant dans la lumière du RER. La protéine mature ne comporte plus cette extension N-terminale.

Les séquences d'adressage peuvent, outre le prépeptide, aussi comporter un signal de rétention endoplasmique, consistant en les peptides KDEL, SEKDEL ou HEKDEL. Ces signaux se trouvent normalement à l'extrémité C-terminale de la protéine et subsistent sur la protéine mature. La présence de ce signal a tendance à augmenter les rendements en protéines recombinantes.

Les signaux d'adressage peuvent, outre le prépeptide, comporter aussi un signal d'adressage vacuolaire ou "propeptide". En présence d'un tel signal, après passage dans le RER, la protéine est adressée aux vacuoles des tissus aqueux, par exemple les feuilles, ainsi qu'aux corps protéiques des tissus de réserve, par exemple les graines, tubercules et racines. L'adressage de la protéine vers les corps protéiques de la graine est particulièrement intéressant en raison de la capacité de la graine à accumuler des protéines, jusqu'à 40% des protéines par rapport à la matière sèche, dans des organites cellulaires dérivés des vacuoles, appelés corps protéiques et en raison de la possibilité de stocker plusieurs années les graines contenant les protéines recombinantes à l'état déshydraté.

Comme propeptide, on peut utiliser un signal d'origine animale ou végétale, les signaux végétaux étant particulièrement préférés, par exemple la prosporamine. Le propeptide peut être N-terminal ("N-terminal targeting peptide" ou NTTP), ou C terminal (CTTP) Dans la mesure où les propeptides sont normalement clivés dès l'entrée de la protéine dans la vacuole, il n'est pas présent dans la protéine mature.

L'utilisation du peptide signal ou prépeptide, peut conduire à la glycosylation de la protéine. Normalement, la globine n'a pas de sites de N-glycosylation, mais ceux-ci peuvent être introduits par mutagénèse. Les chaînes α et β peuvent aussi présenter des sites de O-glycosylation.

En l'absence de tout signal d'adressage, la protéine est exprimée dans le cytoplasme.

L'acide nucléique introduit dans la cellule végétale peut aussi comprendre des séquences régulatrices de transcription reconnues par la cellule végétale. L'acide nucléique est alors un "gène chimérique". Les séquences régulatrices comprennent un ou plusieurs promoteur(s) d'origine végétale, virale ou provenant d'Agrobacterium tumefaciens. Il peut s'agir de promoteurs constitutifs, par exemple le 35S du CaMV, le double 35S, les promoteurs Nos ou OCS, ou des promoteurs spécifiques de certains tissus comme le grain ou spécifiques de certaines phases de développement de la plante. Comme promoteurs spécifiques de graines, on peut citer le promoteur du gène de la napin et de l'acyl carrier protéine (ACP) (EP-A-0255378), ainsi que les promoteurs des gènes AT2S d'Arabidopsis thaliana, c'est-à-dire les promoteurs PAT2S1, PAT2S2, PAT2S3 et PAT2S4 (Krebbers et al., Plant Physiol., 1988, vol. 87, pages 859-866). Il est particulièrement préféré d'utiliser le promoteur de la cruciférine ou de la phaséoline ou pGEA1 et pGEA6 d'Arabidopsis, promoteurs de gènes de type "Em, Early Methionine labelled protein" fortement exprimée au cours des phases de dessication de la graine.

Il peut être envisagé d'utiliser des "enhancers" pour améliorer l'efficacité d'expression. Lorsque la transformation a lieu directement dans les génomes chloroplastiques et mitochondriaux, des promoteurs de gènes spécifiques de ces compartiments peuvent être utilisés.

Les séquences régulatrices de transcription comprennent normalement des séquences de terminaison de transcription qui sont d'origine végétale ou virale, par exemple 35S, ou bactérienne (Agrobacterium).

Lorsque l'acide nucléique transformant ne comprend pas de séquences régulatrices, il est préférable d'ajouter sur chaque extrémité de l'acide nucléique, une séquence d'ADN homologue aux séquences génomiques qui jouxtent un site d'insertion particulier dans le génome. Ceci permet l'intégration du construit par recombinaison homologue, à un endroit où des séquences régulatrices endogènes peuvent contrôler l'expression des séquences hétérologues.

Les acides nucléiques de l'invention peuvent également comprendre un ou plusieurs intron(s), de préférence d'origine végétale. Ces introns provenant d'un gène végétal sont introduits artificiellement afin d'augmenter l'efficacité de l'expression de la séquence hétérologue.

En effet, il a été démontré, particulièrement chez les monocotylédones que l'insertion d'un intron dans la partie 5'non traduite d'un gène, c'est-à-dire entre le site d'initiation de transcription et le site d'initiation de traduction, conduit à une amélioration de la stabilité du messager, et par conséquence, à une meilleure expression. Le ou les introns utilisé(s) de cette manière proviennent de préférence d'une monocotylédone telle que le maïs. Il s'agit de préférence, mais pas obligatoirement, du premier intron du gène.

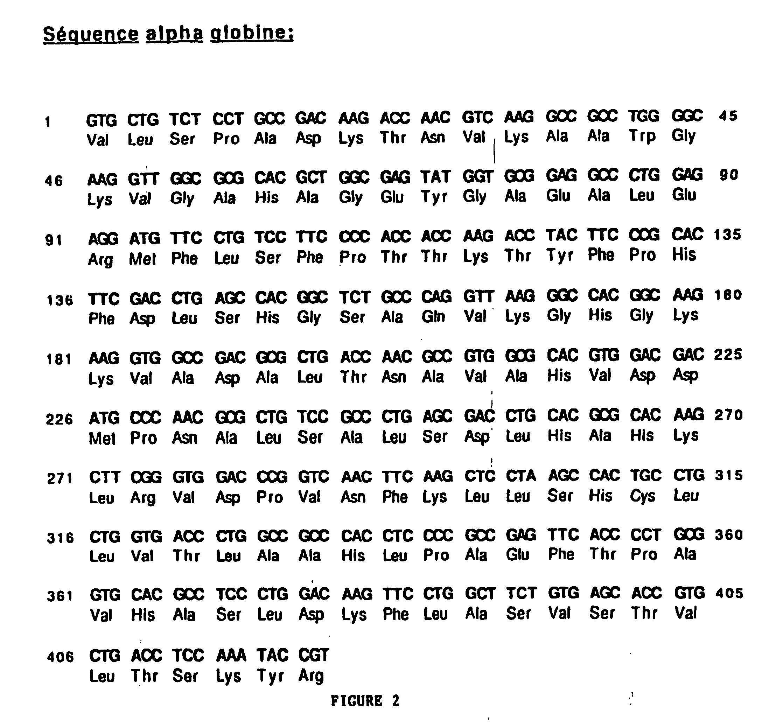

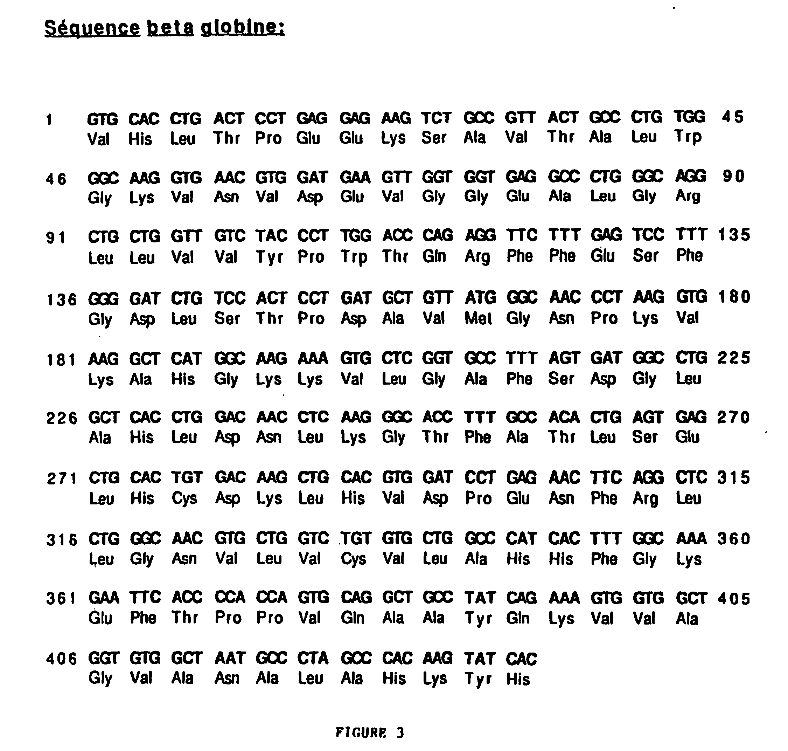

La séquence d'acides nucléiques codant pour l'$\alpha$- et la $\beta$-globine et ses variantes est normalement de l'ADNc. Des séquences appropriées sont illustrées dans les figures 2 et 3, toute séquence dégénérée peut aussi être utilisée ainsi que les séquences des variantes telles que définies ci-dessus.

L'introduction de la ou des molécule(s) d'acides nucléiques dans la cellule végétale peut s'effectuer de manière stable soit par transformation du génome nucléaire, soit par transformation du génome chloroplastique de la cellule végétale, soit par transformation du génome mitochondrial.

Pour la transformation du génome nucléaire, des techniques classiques peuvent être mises en oeuvre. Tous les moyens connus pour introduire de l'ADN étranger dans les cellules végétales peuvent être utilisés, par exemple Agrobacterium, électroporation, fusion de protoplastes, bombardement avec canon à particules, ou pénétration d'ADN dans des cellules comme le pollen, la microspore, la graine et l'embryon immature. Les vecteurs viraux tels que les Geminivirus ou les virus satellites peuvent également être utilisés comme moyens d'introduction. Agrobacterium tumefaciens et rhizogenes constituent le moyen préféré. Dans ce cas, la séquence de l'invention est introduite dans un vecteur approprié avec toutes les séquences régulatrices nécessaires telles que promoteurs, terminateurs, etc... ainsi que toute séquence nécessaire pour sélectionner les transformants ayant intégré les séquences hétérologues.

La transformation du génome nucléaire de la cellule végétale est souvent effectuée en utilisant les signaux d'adressage mentionnés ci-dessus et qui déterminent le compartiment cellulaire où se fera l'expression et/ou l'accumulation de la protéine.

Selon une autre variante de l'invention, l'introduction de l'acide nucléique dans la cellule végétale peut être effectuée par la transformation des génomes mitochondriaux ou chloroplastiques (voir par exemple Carrer et al., Mol. Gen. Genet., 1993, 241, 49-56).

Les techniques de transformation directe des chloroplastes ou mitochondries sont connues en soi et peuvent comprendre les étapes suivantes :

i) introduction de l'ADN transformant par la technique biolistique (Svab et al., P.N.A.S., 1990, 87, 8526-8530) ;
ii) intégration de l'ADN transformant par deux événements de recombinaison homologue ;
iii) élimination sélective des copies du génome sauvage au cours de divisions cellulaires répétées sur milieu sélectif.

Afin de permettre la recombinaison homologue de l'ADN transformant, deux fragments d'ADN homologues aux séquences génomiques, par exemple les gènes rbcL et ORF 512 sont ajoutés à chaque extrémité de l'ADN à insérer dans le génome.

La transformation directe des chloroplastes ou mitochondries présente l'avantage d'augmenter sensiblement le rendement en hémoglobine mais la méthionine N-terminale est retenue.

Selon une autre variante de l'invention, l'acide nucléique hétérologue peut être introduit dans la cellule végétale par l'intermédiaire d'un vecteur viral.

Le procédé de l'invention comprend une étape de détection des protéines héminiques et notamment de l'hémoglobine et de ses dérivés. Ceci permet de vérifier si la plante ou la cellule végétale est capable non seulement d'exprimer les protéines hétérologues, mais aussi de les assembler correctement avec le noyau porphyrique. Pour l'hémoglobine dans un environnement complexe contenant d'autres chromophores ou des molécules diffusant la lumière, on utilisera avantageusement la détection par spectroscopie optique résolue dans le temps. Cette technique est décrite en détail dans les exemples. D'autres techniques de détection consistent en l'utilisation d'anticorps spécifiques pour les chaînes alpha ou bêta globine, ou leurs variantes. Les techniques spectrométriques et immunologiques peuvent être utilisées en association l'une avec l'autre. La mise en oeuvre de ces techniques permet de sélectionner les plantes qui sont capables de produire l'hémoglobine et ses dérivés selon l'invention.

Le procédé de l'invention comprend en outre une étape de récupération ou d'extraction de l'hémoglobine ou de ses dérivés des tissus végétaux. L'extraction est normalement faite par broyage des tissus, par exemple feuilles ou grains, dans un tampon approprié, filtrage du broyat, précipitation des protéines dans le surnageant, centrifugation et reprise du culot dans un tampon approprié avec dialyse. Une étape de purification partielle peut aussi être effectuée à ce stade par chromatographie sur colonne d'échangeuse d'anions.

Le tétramère d'hémoglobine, ou de ses dérivés, est purifié par deux chromatographies successives sur résine échangeuse d'ions suivies par une étape de concentration et saturation du concentrat en monoxyde de carbone. Ces techniques sont décrites en détail dans les exemples.

Lorsque l'expression de l'hémoglobine et de ses dérivés a lieu sous le contrôle d'un promoteur constitutif, tel que le promoteur double 35S, un taux d'expression d'au moins 1% d'hémoglobine par rapport aux protéines totales peut être obtenu. Les protéines représentent environ 10% de la masse sèche de la feuille et une tonne de feuilles sèches de tabac est récoltée par hectare. Il est donc possible d'obtenir de l'ordre de 100 grammes d'hémoglobine par hectare de tabac cultivé en supposant purifier seulement 10% de l'hémoglobine produite.

Le procédé de l'invention permet donc la production d'hémoglobine à des coûts très faibles avec une capacité de production plus élevée que celle obtenue en utilisant des fermenteurs pour la culture de bactéries ou de levure.

Outre le procédé de transformation, l'invention englobe aussi des vecteurs comprenant un ou plusieurs acide(s) nucléique(s) ou gène(s) chimérique(s) définis ci-dessus. Comme exemple de vecteurs, on peut citer des vecteurs binaires ou des plasmides, des vecteurs viraux tels que les geminivirus ou les CaMV.

L'invention concerne également les cellules végétales transformées avec les séquences d'acides nucléiques de l'invention. De préférence, il s'agit de cellules végétales transformées capables de produire une ou plusieurs hémoglobine(s) ou dérivés de l'hémoglobine selon l'invention.

Il peut s'agir de cultures de cellules végétales in vitro, par exemple en milieu liquide. Différents modes de culture ("batch", "fed batch" ou en continu) pour ce type de cellules sont actuellement à l'étude. Les cultures en "batch" sont comparables à celles effectuées en erlenmeyer dans la mesure où le milieu n'est pas renouvelé, les cellules ne disposent ainsi que d'une quantité limitée d'éléments nutritifs. La culture en "fed batch" correspond quant à elle à une culture en "batch" avec une alimentation programmée en substrat. Pour une culture en continu, les cellules sont alimentées en permanence avec du milieu nutritif. Un volume égal du mélange biomasse-milieu est ôté afin de maintenir le volume du réacteur constant. Les quantités de biomasse végétale envisageables avec des cultures en bioréacteurs sont variables selon l'espèce végétale, le mode de culture et le type de bioréacteur. Dans certaines conditions, des densités de biomasse d'environ 10 à 30 g de poids sec par litre de culture peuvent être obtenus, pour des espèces comme Nicotiana tabacum, Vinca rosea et Catharanthus roseus.

Les cellules de l'invention peuvent aussi être immobilisées, ce qui permet d'obtenir une production constante et prolongée de l'hémoglobine. La séparation de l'hémoglobine et la biomasse végétale est aussi facilitée. Comme méthode d'immobilisation, on peut citer l'immobilisation en billes d'alginate, d'agar, à l'intérieur de mousse de polyuréthane, ou bien encore dans des fibres creuses.

Les cellules de l'invention peuvent également être des cultures de racines. Les racines cultivées in vitro, en milieu liquide, sont nommées "Hairy roots", ce sont des racines transformées par la bactérie Agrobacterium rhizogenes.

Au lieu de produire l'hémoglobine de l'invention par culture de cellules végétales, on peut régénérer des plantes chimériques ou transgéniques à partir d'explants transformés, en ayant recours à des techniques connues en soi.

Comme plantes appropriées, on peut citer les Angiospermes comprenant les monocotylédones et les dicotylédons. Plus particulièrement, on peut citer le tabac, les espèces appartenant aux familles botaniques telles que les légumineuses (par exemple les haricots, pois, etc...), les crucifères (par exemple les choux, radis, colza, etc...), les solanacées (par exemple les tomates, pommes de terre, etc...), les cucurbitacées (par exemple le melon), les chénopodiacées (par exemple la betterave potagère), les ombellifères (par exemple les carottes, céleris, etc...). On peut également citer les céréales telles que le blé, le maïs, l'orge, le triticale et le riz, et les oléagineux tels que le tournesol et le soja. Le tabac, la pomme de terre, la tomate et le maïs sont particulièrement préférés. Pour la pomme de terre, l'expression a lieu de préférence dans les tubercules.

L'invention concerne également les graines des plantes transgéniques capables de produire l'hémoglobine ainsi que leurs descendances.

L'invention vise également les protéines héminiques susceptibles d'être obtenues par le procédé de l'invention, en particulier les protéines héminiques capables de fixer de manière réversible l'oxygène, par exemple les hémoglobines et dérivés de celles-ci.

Les hémoglobines de l'invention sont capables de fixer l'$O_2$ de façon réversible avec une affinité ($P_{50}$) de préférence proche des valeurs physiologies (37°C), pH 7,40). L'affinité de la molécule pour l'$O_2$ est exprimée en $P_{50}$ : c'est-à-dire la pression partielle d'$O_2$ lorsque l'hémoglobine ou ses dérivés est saturée à 50%. La $P_{50}$ est mesurée selon les techniques habituelles, par exemple au moyen d'un analyseur qui mesure le pourcentage de saturation en $O_2$ en fonction de la pression en $O_2$ (Kister et al., 1987). Normalement, les hémoglobines de l'invention présentent une vitesse d'auto-oxydation acceptable pour minimiser la formation de méthémoglobine impropre au transport de l'$O_2$. Cette caractéristique peut être mesurée par le spectre d'absorption.

De préférence, les hémoglobines de l'invention sont des tétramères, de préférence alpha$_2$ bâta$_2$, bâta$_4$, ou éventuellement des tétramères de sous-unités chimériques $\alpha/\beta$ (Dumoulin et al., 1994, Art. Cells, Blood Subst., and Immob. Biotech., 22, 733-738) ou des multiples de 4 sous-unités. La taille physique du complexe doit être au moins celle du tétramère afin d'éviter sa filtration par les reins.

Les protéines héminiques de l'invention peuvent être utilisées dans de nombreuses applications pharmaceutiques, cosmétiques ou industrielles. L'invention concerne en particulier des compositions pharmaceutiques comprenant une ou plusieurs protéine(s) héminique(s) selon l'une quelconque des revendications 15 à 23 en association avec un excipient acceptable du point de vue physiologique.

Dans le domaine pharmaceutique, toutes les conditions nécessitant une amélioration du transport de l'oxygène peuvent être traitées par les hémoglobines de l'invention, ces conditions comprennent les suivantes :

- hémorragies aigües ou chroniques,
- états de chocs,
- angioplasties coronariennes ou sylviennes,
- traitements des tumeurs solides, sensibilisation à la gamma-thérapie,
- conservation d'organes avant greffe et pendant le transport,
- hémopathies malignes.

Les hémoglobines de l'invention sont normalement utilisées sous forme d'injection dans des solutions éventuellement stabilisées en ce qui concerne la forme tétramérique du complexe (par exemple, addition de pyridoxal phosphate ou diaspirine) en ce qui concerne l'auto-oxydation. Il est également possible d'utiliser des suspensions d'hémoglobine greffée sur un support afin d'augmenter la durée de vie dans la circulation. Le support peut être tout support conventionnel dans ce domaine, par exemple les polysaccharides.

Différents aspects de l'invention sont illustrés dans les figures :

- Figure 1 : Protoporphyrine III (IX) à fer,
- Figure 2 : Séquence du cDNA de l'$\alpha$-globine humaine (423 paires de bases), et protéine correspondante,
- Figure 3 : Séquence du cDNA de la $\beta$-globine humaine (438 paires de bases), et protéine correspondante,
- Figure 4 : Dispositif expérimental pour photolyse éclair. Un laser à impulsion sert pour la photodissociation des ligands de l'Hb : (HbCO → Hb + CO). Un deuxième faisceau optique, orienté à 90°, détecte les changements d'absorption en fonction de temps après dissociation.
- Figure 5 : Cinétiques de recombinaison bimoléculaire du CO à l'hémoglobine dans l'extrait de plante. Les deux phases correspondent aux deux états allostériques de l'Hb : R (rapide) et T (lente). Conditions : 0.1 atm CO, pH 6-6, 25°C, environ 50% dissociation, 0-1, 1 et 10 µM Hb.
- Figure 6 : cinétiques de recombinaison du CO à l'hémoglobine en fonction du pourcentage de dissociation (par variation de l'énergie laser). Les cinétiques sont sensibles au nombre de ligands dissociés (1 à 4). A fort niveau de dissociation, l'Hb (désoxy ou mono-ligandée) bascule vers la forme lente "T". A faible énergie laser, les tétramères (principalement avec trois ligands) restent dans la forme rapide "R".
- Figure 7 : Analyse par western blot de l'extrait des graines du tabac transgénique T26-22 transformé par le plasmide pBIOC59. Les extraits de graines (75 µg de protéines) d'un tabac non-transformé (1) et d'un tabac transgénique (T26-22) (2), des marqueurs de poids moléculaire (3) et de l'HbA (50 ng) (4) sont séparés en électrophorèse SDS-PAGE 17% en conditions réductrices. Le western blot est réalisé dans les conditions décrites au chapitre X. a. Les marqueurs de poids moléculaire et les globines $\alpha$ et $\beta$ sont indiqués.

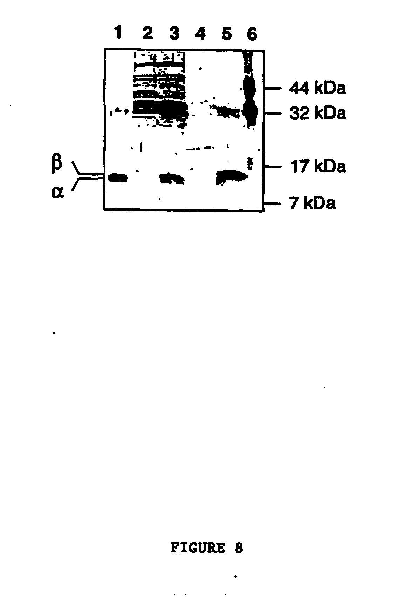

- Figure 8 : Analyse par western blot des fractions obtenues lors de la purification partielle. Les protéines des fractions éluées de la Sephacryl S-100 (37 µg), de la S-Sepharose (30 µg) obtenues pendant la purification à partir des mélanges de graines témoins [respectivement (2) et FE-Témoin (4)] et de graines accumulant la rHb [respectivement (3) et FE-rHb (5)], de l'HbA (50 ng) (1) et des marqueurs de poids moléculaire (6) ont été séparés en électrophorèse SDS-PAGE 17% en conditions réductrices. Le western blot est effectué dans les conditions décrites dans le chapitre X.a. Les marqueurs de poids moléculaires et les globines $\alpha$ et $\beta$ sont indiqués.

- Figure 9 : Cinétique de recombinaison du CO à la fraction FE-rHb. La cinétique après photolyse éclair est caractéristique de l'Hb tétramèrique normale. La fraction FE-Témoin provenant des plantes de contrôle donne un signal d'amplitude de 1 mDO soit environ 50 fois plus faible que celle observée pour la fraction FE-rHb (48 mDO).
- Figure 10 : Cinétique de recombinaison du CO à la fraction FE-rHb à différents niveaux d'intensité de laser. Des résultats similaires sont observés pour l'HbA (figure 6).
- Figure 11 : Démonstration de la liaison réversible de l'oxygène à l'échantillon FE-rHb. Comme les échantillons d'oxyhémoglobine ne donnent que des signaux faibles, nous avons utilisé pour ces mesures la technique du mélange d'une atmosphère CO et d'$O_2$. Après photodissociation du CO, la phase rapide correspond à la liaison de l'oxygène. L'oxygène est ensuite remplacé par le CO qui peut être photodissocié à nouveau. La figure montre également les cinétiques de recombinaison du CO du même échantillon équilibré sous 1 atm ou 0.1 atm CO.

## EXEMPLES

## I. CONSTRUCTION DE PLASMIDES BINAIRES D'EXPRESSION DE BASE PERMETTANT LA PRODUCTION DE PROTEINES RECOMBINANTES DANS LES FEUILLES DE TABAC.

L'expression de gènes dans les feuilles de tabac nécessite les séquences régulatrices suivantes :

1) le promoteur constitutif double 35S (pd35S) du CaMV (virus de la mosaïque du chou-fleur). Il correspond à une duplication des séquences activant la transcription situées en amont de l'élément TATA du promoteur 35S naturel (Kay et al., 1987).
2) la séquence terminatrice de transcription, terminateur polyA 35S, qui correspond à la région en 3' non codante de la séquence du virus à ADN bicaténaire circulaire de la mosaïque du chou-fleur produisant le transcrit 35S (Franck et al., 1980).

Les constructions des différents plasmides via l'utilisation de techniques d'ADN recombinant (Sambrook et al., 1989) dérivent de pBIOC4. Ce plasmide binaire dérive de pGA492 (An, 1986) qui contient entre les bordures droite et gauche, issues du plasmide pTiT37 d'<u>Agrobacterium tumefaciens</u>, sur son ADN de transfert, les séquences suivantes :

- le promoteur constitutif du gène nos codant pour la nopaline synthase (Depicker et al., 1982),
- la séquence codante du gène nptII codant pour la néomycine phosphotransférase II (Berg et Berg, 1983) délétée de la région des 8 premiers codons dont le codon initiateur méthionine ATG et fusionnée à la séquence des 14 premiers codons de la séquence codante du gène nos (Depicker et al., 1982), la séquence codante du gène nos dépourvue de la région des 14 premiers codons, le terminateur nos (Depicker et al., 1982), un polylinker (HindIII-XbaI-SacI-HpaI-KpnI-ClaI-BglII) précédant le gène cat codant pour la chloramphénicol acétyltransférase (Close et Rodriguez, 1982) et les séquences terminatrices du gène 6 du plasmide pTiA6 d'<u>Agrobacterium tumefaciens</u> (Liu et al., 1993).

Pour éliminer la quasi-totalité de la séquence codante du gène cat, le plasmide pGA492 a été doublement digéré par SacI (site de restriction du polylinker) et par ScaI (site de restriction présent dans la séquence du gène cat) puis soumis à l'action de l'enzyme T4 DNA polymérase (New England Biolabs) selon les recommandations du fabricant. La ligation du plasmide modifié (20 ng) a été réalisée dans un milieu réactionnel de 10 μl contenant 1 μl de tampon T4 DNA ligase x 10 (Amersham) et 2,5 U d'enzyme T4 DNA ligase (Amersham) à 14°C pendant 16 heures. Les bactéries, <u>E. coli</u> DH5α rendues préalablement compétentes, ont été transformées (Hanahan, 1983).
L'ADN plasmidique des clones obtenus, sélectionnés sur 12 μg/ml tétracycline, a été extrait selon la méthode de la lyse alcaline (Birnboim et Doly, 1979) et analysé par digestion enzymatique par des enzymes de restriction. Puis, le site de restriction HindIII de l'ADN plasmidique du clone retenu a été modifié en un site de restriction EcoRI à l'aide d'un adaptateur HindIII - EcoRI phosphorylé (Stratagene Cloning Systems). Pour réaliser cette modification, 500 ng d'ADN plasmidique du clone retenu ont été digérés par HindIII, déphosphorylés par l'enzyme phosphatase alcaline d'intestin de veau (Boehringer Mannheim) selon les recommandations du fabricant et coprécipités en présence de 1500 ng d'ADN adaptateur HindIII-EcoRI, 1/10 volume d'acétate de sodium 3M pH4,8 et 2,5 volumes d'éthanol absolu à -80°C pendant 30 min. Après centrifugation à 12000g pendant 30 min., l'ADN précipité a été lavé à l'éthanol 70%, séché, repris dans 8 μl d'eau, porté à 65°C pendant 10 min., puis ligué en présence de 1 μl de tampon T4 DNA ligase x 10 (Amersham) et 2,5 U d'enzyme T4 DNA ligase (Amersham) à 14°C pendant 16 heures. Après inactivation de la T4 DNA ligase à 65°C pendant 10 min., le mélange réactionnel de ligation a été digéré par EcoRI, purifié par électrophorèse sur gel d'agarose 0,8%, electroélué (Sambrook et al., 1989), précipité en présence de 1/10 de volume de 3M acétate de sodium pH4,8 et de 2,5 volumes d'éthanol absolu à -80°C pendant 30 min., centrifugé à 12000g pendant 30 min., lavé à l'éthanol 70%, puis séché. Les bactéries, E. coli DH5α rendues préalablement compétentes, ont été transformées (Hanahan, 1983). L'ADN plasmidique des clones obtenus, sélectionnés sur 12 µg/ml tétracycline, a été extrait selon la méthode de la lyse alcaline (Birnboim et Doly, 1979) et analysé par digestion enzymatique par HindIII et EcoRI notamment. Le plasmide binaire résultant, qui ne possède plus que les 9 derniers codons de la séquences codante du gène cat et dont le site EcoRI est unique, a été appelé pBIOC4.

a. CONSTRUCTION DU PLASMIDE BINAIRE D'EXPRESSION pBIOC21.

La cassette d'expression, constituée du promoteur pd35S et du terminateur polyA 35S, a été isolée à partir du plasmide pJIT163D. Le plasmide pJIT163D dérive du plasmide pJIT163 qui dérive lui-même du plasmide pJIT60 (Guerineau et Mullineaux, 1993). Le plasmide pJIT163 possède un codon ATG entre les sites HindIII et SalI du polylinker. Pour supprimer cet ATG et obtenir le plasmide pJIT163D, l'ADN plasmidique pJIT163 a été digéré doublement par HindIII et SalI, purifié par électrophorèse sur gel d'agarose 0,8%, électroélué (Sambrook et al., 1989), précipité en présence de 1/10 de volume de 3M acétate de sodium pH4,8 et de 2,5 volumes d'éthanol absolu à -80°C pendant 30 min., centrifugé à 12000g pendant 30 min., lavé à l'éthanol 70%, séché, soumis à l'action de l'enzyme Klenow (New England Biolabs) selon les recommandations du fabricant, déprotéinisé par extraction avec 1 volume de phénol:chloroforme:alcool isoamylique (25:24:1) puis 1 volume de chloroforme:alcool isoamylique (24:1), précipité en présence de 1/10 de volume de 3M acétate de sodium pH4,8 et de 2,5 volumes d'éthanol absolu à -80°C pendant 30 min., centrifugé à 12000g pendant 30 min., lavé à l'éthanol 70%, séché, et enfin, ligué en présence de 1 µl de tampon T4 DNA ligase x 10 (Amersham) et 2,5 U d'enzyme T4 DNA ligase (Amersham) à 14°C pendant 16 heures. Les bactéries, E. coli DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Pour isoler la cassette d'expression constituée du promoteur pd35S et du terminateur polyA 35S (fragment SacI-XhoI), l'ADN plasmidique du clone pJIT163D retenu a été digéré par SacI et XhoI. Le fragment SacI-XhoI, portant la cassette d'expression, a été purifié par électrophorèse sur gel d'agarose 0,8%, électroélué (Sambrook et al., 1989), précipité en présence de 1/10 de volume de 3M acétate de sodium pH4,8 et de 2,5 volumes d'éthanol absolu à -80°C pendant 30 min., centrifugé à 12000g pendant 30 min., lavé à l'éthanol 70%, séché, puis soumis à l'action de l'enzyme Mung Bean nucléase (New England Biolabs) selon les recommandations du fabricant. Cet insert purifié (200 ng) a été cloné dans l'ADN plasmidique de pBIOC4 (20 ng) digéré par EcoRI, traité par l'enzyme Mung Bean nucléase et déphosphorylé par l'enzyme phosphatase alcaline d'intestin de veau (Boehringer Mannheim) selon les recommandations du fabricant. La réaction de ligation a été effectuée dans 20 µl en présence de 2 µl de tampon T4 DNA ligase x 10 (Amersham), de 2 µl de 50% polyéthylène glycol 8000 et de 5 U d'enzyme T4 DNA ligase (Amersham) à 14°C pendant 16 heures. Les bactéries, E. coli DH5α rendues préalablement compétentes, ont été transformées (Hanahan, 1983). L'ADN plasmidique des clones obtenus, sélectionnés sur 12 µg/ml tétracycline, a été extrait selon la méthode de la lyse alcaline (Birnboim et Doly, 1979) et analysé par digestion enzymatique par des enzymes de restriction. Le plasmide résultant a été appelé pBIOC21.

b. CONSTRUCTION DU PLASMIDE BINAIRE DE CO-EXPRESSION pBIOC43.

Le plasmide binaire de co-expression permettra une expression de deux gènes dans le même vecteur binaire.
Le plasmide binaire de co-expression dérive de pBIOC21. Il contient deux cassettes d'expression constituées chacune d'un promoteur pd35S et d'un terminateur polyA 35S mais différent par le polylinker séparant le promoteur du terminateur. L'une des cassettes d'expression est celle de pBIOC21 déjà décrite au paragraphe I. a. L'autre cassette d'expression a été obtenue en remplaçant le polylinker HindIII-BamHI-SmaI-EcoRI de pJIT163D (décrit au paragraphe I. a.) par un adaptateur HindIII-EcoRI portant les sites de restriction PacI, AscI, MluI et HpaI. Cet adaptateur a été obtenu par renaturation des 2 oligodésoxynucléotides WD11 (5' AGC TGA TTA ATT AAG GCG CGC CAC GCG TTA AC 3') et WD12 (5' AAT TGT TAA CGC GTG GCG CGC CTT AAT TAA TC 3') qui sont complémentaires pour leurs 28 nucléotides 3' terminaux. Cent µM de chacun de ces deux oligodésoxynucléotides ont été préalablement phosphorylés par action de 10 U d'enzyme T4 polynucléotide kinase (New England Biolabs) dans un volume réactionnel total de 10 µl contenant 1 µl de tampon T4 polynucléotide kinase x 10 (New England Biolabs) et 3 µl d'ATP (95 mM). Les deux mélanges réactionnels ont été incubés à 37°C pendant 1 heure, puis à 65°C pendant 20 min. Ils ont été ensuite rassemblés et leur volume a été complété à 500 µl. Après extraction avec 1 volume de phénol:chloroforme:alcool isoamylique (25:24:1) et 1 volume de chloroforme:alcool isoamylique (24:1), 50 µl de 3M acétate de sodium pH6.0 ont été ajoutés. Le mélange réactionnel a été incubé à 80°C pendant 10 min., puis refroidi lentement à température ambiante. L'ADN a ensuite été précipité en présence de 2,5 volumes d'éthanol absolu à -80°C pendant 30 min., centrifugé à 14000g à 4°C pendant 1 heure, lavé à l'éthanol 70%, centrifugé à 14000g à 4°C pendant 10 min., séché, repris dans 10 µl de H20. Le fragment d'ADN HindIII-EcoRI a ensuite été cloné aux sites HindIII-EcoRI de l'ADN plasmidique pJIT163D préalablement déphosphorylé par l'enzyme phosphatase alcaline d'intestin de veau (New England Biolabs) selon les recommandations du fabricant. La réaction de ligation a été effectuée dans un volume réactionnel de 20 µl en présence de 1 U de T4 DNA ligase (Gibco-BRL) pour une concentration totale d'ADN de 8,5 nM avec un rapport molaire vecteur/insert de 1 et de 4 µl de tampon T4 DNA ligase x 5 (Gibco-BRL) à 25°C pendant 16 heures. Les bactéries, E. coli DH5α rendues préalablement compétentes, ont été transformées (Hanahan, 1985). L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC42. Sa validité a été vérifiée par séquençage à l'aide du kit "Sequenase Version 2.0 DNA Sequencing" commercialisé par United States Biochemical (USB) selon la méthode des didésoxynucléotides (Sanger et al., 1977). Les conditions réactionnelles suivent les indications du fabricant excepté pour la dénaturation et l'hybridation. Le milieu réactionnel contenant l'ADN plasmidique (0,5 à 1 pmoles), l'amorce oligonucléotidique (2 pmoles), 10% de DMSO et le tampon réactionnel x 1 (USB), est incubé à 100°C pendant 10 min., puis refroidi brutalement à - 80°C dans la carboglace.

A partir de pBIOC42, le fragment d'ADN codant pour la cassette d'expression constituée du promoteur pd35S et du terminateur polyA 35S a été isolé par double digestion par SacI et XhoI. Il a été purifié par électrophorèse sur gel d'agarose 0,75%, puis soumis à l'action du kit "Geneclean II" commercialisé par BIO101 selon les indications du fabricant. Puis, ce fragment d'ADN a été inséré aux sites SacI et XhoI du plasmide pBCSK+ commercialisé par Stratagene et préalablement déphosphorylé par l'enzyme phosphatase alcaline d'intestin de veau (New England Biolabs) selon les recommandations du fabricant. La ligation a été réalisée dans un volume réactionnel de 20 µl en présence de 1 U de T4 DNA ligase (Gibco-BRL) pour une concentration totale d'ADN de 8,5 nM avec un rapport molaire vecteur/insert de 1 et de 4 µl de tampon T4 DNA ligase x 5 (Gibco-BRL) à 25°C pendant 16 heures. Les bactéries, E. coli DH5α rendues préalablement compétentes, ont été transformées (Hanahan, 1985). L'ADN plasmidique des clones obtenus, sélectionnés sur 30 µg/ml de chloramphénicol, a été extrait selon la méthode de la lyse alcaline (Stephen et al., 1990) et analysé par digestion enzymatique par des enzymes de restriction. Le plasmide résultant a été appelé pBIOC75.

A partir de pBIOC75, le fragment d'ADN portant la cassette d'expression constituée du promoteur pd35S et du terminateur polyA 35S a été isolée par digestion par KpnI. Il a été purifié par électrophorèse sur gel d'agarose 0,75%, puis soumis à l'action du kit "Geneclean II" commercialisé par BIO101 selon les indications du fabricant. Puis, ce fragment d'ADN a été ligué à l'ADN plasmidique de pBIOC21 digéré par KpnI et déphosphorylé par l'enzyme phosphatase alcaline d'intestin de veau (New England Biolabs) selon les recommandations du fabricant. La ligation a été réalisée dans un volume réactionnel de 20 µl en présence de 1 U de T4 DNA ligase (Gibco-BRL) pour une concentration totale d'ADN de 8,5 nM avec un rapport molaire vecteur/insert de 1 et de 4 µl de tampon T4 DNA ligase x 5 (Gibco-BRL) à 25°C pendant 16 heures. Les bactéries, E. coli DH5α rendues préalablement compétentes, ont été transformées (Hanahan, 1985). L'ADN plasmidique des clones obtenus, sélectionnés sur 12 µg/ml de tétracycline, a été extrait selon la méthode de la lyse alcaline (Stephen et al., 1990) et analysé par digestion enzymatique par des enzymes de restriction. Le plasmide résultant a été appelé pBIOC43.

**II. CONSTRUCTION DES GENES CHIMERIQUES CODANT POUR LES CHAINES DE GLOBINE, α ET β, PERMETTANT UNE EXPRESSION DE l'HEMOGLOBINE HUMAINE RECOMBINANTE DANS LE CYTOPLASME DES FEUILLES DE TABAC.**

Le plasmide alphalpJW101 renferme le cDNA de la chaîne α de globine cloné dans le plasmide pMB9 comme décrit par Wilson et al. (1978).

Le phage M13mp10 cIIFX bêta renferme le cDNA de la chaîne B de globine cloné dans le phage M13 mp10 comme décrit par Nagai et al. (1985). Dans cette construction, l'ADNc codant pour la B globine a été inséré en 3' de la séquence codante pour la protéine cII du phage lambda, suivie de celle codant pour le tétrapeptide FX, formant un gène de fusion dans lequel le codon initiateur ATG de la β globine a été délété.

a. CONSTRUCTION DU PLASMIDE pBIOC44 CONTENANT LE cDNA CODANT POUR L'α GLOBINE POUR UN ADRESSAGE CYTOPLASMIQUE.

Pour obtenir un adressage cytoplasmique de la chaîne α de globine, le codon méthionine initiateur de la chaîne α globine a été maintenu.

Le cDNA codant pour la chalne α globine à adressage cytoplasmique a été obtenu en trois étapes. Les deux premières étapes ont permis de supprimer le site interne HindIII (substitution d'un T en un C) en position 276 de la séquence codante tandis que la troisième étape réunit les 2 fragments du cDNA codant pour la chaîne α globine recombinante.

La première étape a consisté en l'amplification des 95 premiers codons de la chalne α globine mature sur le plasmide alphalpJW101 à l'aide des 2 oligodésoxynucléotides, WD13 (5' tacaagcttaaca ATG GTG CTG TCT CCg GCC GAC 3') et AD27 (5' CGG GTC CAC CCG GAG CTT GTG 3'). L'amorce WD13 apporte le site de restriction HindIII, la séquence aaca favorisant l'initiation de la traduction (Joshi, 1987) et précédant le codon initiateur ATG suivi des 6 premiers codons de la chaîne α globine mature dont le quatrième (CCT) est substitué en CCg (mutation silencieuse) pour créer le site de restriction EagI. L'amorce AD27 permet la suppression du site de restriction HindIII par substitution du nucléotide T en C (position 276 de la séquence codante). L'amplification PCR a été réalisée dans 100 µl de milieu réactionnel contenant 10 µl de tampon Taq DNA polymérase x 10 (100 mM Tris-HCl pH8,4, 500 mM KCl et 20 mM MgCl2), 16 µl du mélange de dNTP (1,25 mM dATP, 1,25 mM dCTP, 1,25 mM dGTP et 1,25 mM dTTP), 10 µl de chacune des amorces décrites ci-dessus à 10 µM, 10 µl d'ADN matrice (alphalpJW101) à 1 ng/µl et 0,5 µl de Taq DNA polymérase à 5 U/µl (Perkin Elmer). Trente cycles comportant chacun 30 sec. de dénaturation à 97°C, 1 min. d'hybridation à 55°C et 2 min. d'élongation à 72°C ont été effectués dans l'appareil "Crocodile II" d'Appligène. Les fragments d'ADN amplifiés ont ensuite été purifiés par électrophorèse sur gel d'agarose 1,8% et action du kit "Geneclean II" commercialisé par BIO101 selon les indications du fabricant. Les fragments d'ADN amplifiés purifiés sont repris dans 20 µl.

La deuxième étape a consisté en l'amplification des 54 derniers codons de la chaîne α globine mature sur le plasmide alphalpJW101 à l'aide des 2 oligodésoxynucléotides, AD26 (5' CAC AAG CTC CGG GTG GAC CCG 3') et WD14 (5' gcgaattc TCA ACG GTA TTT GGA GGT CAG CAC 3'). L'amorce WD14 apporte le site de restriction EcoRI situé juste après le codon stop. L'amorce AD26 permet la suppression du site de restriction HindIII par substitution du nucléotide T en C (position 276 de la séquence codante). L'amplification PCR a été réalisée comme décrite dans la première étape. Le traitement des fragments d'ADN amplifiés a été effectué comme décrit dans la première étape.

La troisième étape a été l'amplification par PCR du cDNA complet codant pour la chaîne α globine (142 codons dont l'ATG initiateur). Les deux types de fragments d'ADN amplifiés dans les première et deuxième étapes ont servi d'ADN matrice et les deux amorces utilisées ont été WD13 et WD14. L'amplification PCR a été réalisée comme décrite dans la première étape excepté que la température d'hybridation du cycle est de 60°C. Les fragments d'ADN amplifiés ont ensuite été extraits par de l'éther saturé en $H_2O$ après avoir ajusté le volume à 500 µl avec du tampon TE (10 mM Tris-HCl pH8,0, 1 mM EDTA). Après extraction avec 1 volume de phénol:chloroforme:alcool isoamylique (25:24:1) et 1 volume de chloroforme:alcool isoamylique (24:1), les fragments d'ADN ont été précipités en présence de 1/10 de volume de 3M acétate de sodium pH6,0 et de 2 volumes d'éthanol absolu à - 80°C pendant 30 min., centrifugé à 14000g à 4°C pendant 30 min., lavé à l'éthanol 70%, centrifugé à 14000g à 4°C pendant 10 min., séché, repris dans 50 µl de $H_2O$. Puis, 25 µl de ces fragments d'ADN ont été digérés doublement par HindIII et EcoRI, purifiés par électrophorèse sur gel d'agarose 1,8% et par action du kit "Geneclean II" (BIO101) et clonés aux sites HindIII et EcoRI du plasmide pNEB193 commercialisé par New England Biolabs, et préalablement déphosphorylé par l'enzyme phosphatase alcaline d'intestin de veau (New England Biolabs) selon les recommandations du fabricant. La ligation et la transformation ont été réalisées comme décrites au chapitre I. b. L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC44. La séquence nucléotidique du cDNA codant pour la chaîne α globine recombinante a été vérifiée par séquençage à l'aide du kit "Sequenase Version 2.0 DNA Sequencing" commercialisé par United States Biochemical (USB) comme décrit au chapitre I. b. Le séquençage a révélé deux mutations silencieuses situées au quarante-huitième nucléotide (C modifié en T) et au cinquante-quatrième (T modifié en C) de la séquence codante pour la chaîne α globine.

## b. CONSTRUCTION DU PLASMIDE pBIOC45 CONTENANT LE cDNA CODANT POUR LA β GLOBINE POUR UN ADRESSAGE CYTOPLASMIQUE.

Pour obtenir un adressage cytoplasmique de la chaîne β de globine, le codon méthionine a été fusionné au premier codon de la chaîne β globine mature en maintenant la phase ouverte de lecture puisque l'ATG avait été délété dans la construction M13mp10 cIIFX bêta.

Le cDNA codant pour la chaîne β globine à adressage cytoplasmique a été obtenu par amplification PCR des 146 codons constituant la chaîne β globine mature sur le phage M13mp10 cIIFX bêta à l'aide des 2 oligodésoxynucléotides, WD15 (5' gtcattaattaaca ATG GTG CAC CTG ACT CCT GAG GAG AAG TCg GCC GTT AC 3') et WD16 (5' aatgagctcgttaacgcgt TTA GTG ATA CTT GTG GGC CAG GGC 3'). L'amorce WD15 apporte le site de restriction PacI, la séquence aaca favorisant l'initiation de la traduction (Joshi, 1987) et le codon initiateur ATG suivi des 12 premiers codons de la chaîne β globine mature dont le neuvième (TCT) est substitué en TCg (mutation silencieuse) pour créer le site de restriction EagI. L'amorce WD16 apporte les sites de restriction MluI, HpaI et SacI placés après le codon stop. L'amplification PCR et le traitement des fragments d'ADN amplifiés ont été réalisées comme décrits dans la troisième étape du chapitre II. a. Puis, 25 µl de ces fragments d'ADN ont été digérés doublement par PacI et SacI, purifiés par électrophorèse sur gel d'agarose 1,8% et par action du kit "Geneclean II" (BIO101) et clonés aux sites PacI et SacI du plasmide pNEB193 commercialisé par New England Biolabs, préalablement déphosphorylé par l'enzyme phosphatase alcaline d'intestin de veau (New England Biolabs) selon les recommandations du fabricant. La ligation et la transformation ont été réalisées comme décrites au chapitre I. b. L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC45. La séquence nucléotidique du cDNA codant pour la chaîne β globine recombinante a été vérifiée par séquençage comme décrit au chapitre I. b.

c. CONSTRUCTION DES PLASMIDES BINAIRES d'EXPRESSION, pBIOC 46 ET pBIOC47, ET DU PLASMIDE BINAIRE DE CO-EXPRESSION pBIOC49 POUR L'ADRESSAGE CYTOPLASMIQUE.

c.1. CONSTRUCTION DU PLASMIDE BINAIRE pBIOC46 CONTENANT LE cDNA CODANT POUR L'a GLOBINE POUR UN ADRESSAGE CYTOPLASMIQUE.

A partir de pBIOC44, le fragment HindIII-EcoRI portant le cDNA codant pour la chaîne α globine à adressage cytoplasmique a été isolé par double digestion enzymatique par HindIII et EcoRI, purifié par électrophorèse sur gel d'agarose 1,8% et par action du kit "Geneclean II" (BIO101). Puis, ce fragment d'ADN a été ligué à l'ADN plasmidique de pBIOC21 doublement digéré par HindIII et EcoRI, et déphosphorylé par l'enzyme phosphatase alcaline d'intestin de veau (New England Biolabs) selon les recommandations du fabricant. La ligation et la transformation ont été réalisées comme décrites au chapitre I. b. L'ADN plasmidique des clones obtenus, sélectionnés sur 10 µg/ml tétracycline, a été extrait selon la méthode de la lyse alcaline (Stephen et al., 1990) et analysé par digestion enzymatique. Le clone résultant a été appelé pBIOC46. La séquence nucléotidique du cDNA codant pour la chaîne a globine recombinante a été vérifiée par séquençage comme décrit au chapitre I. b. L'ADN plasmidique du vecteur binaire pBIOC46 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

c.2. CONSTRUCTION DU PLASMIDE BINAIRE pBIOC47 CONTENANT LE cDNA CODANT POUR LA β GLOBINE POUR UN ADRESSAGE CYTOPLASMIQUE.

A partir de pBIOC45, le fragment HindIII-EcoRI portant le cDNA codant pour la chaîne β globine à adressage cytoplasmique a été isolé par double digestion enzymatique par HindIII (digestion totale) et EcoRI (digestion partielle), purifié par électrophorèse sur gel d'agarose 1,8% et par action du kit "Geneclean II" (BIO101). Puis, ce fragment d'ADN a été ligué à l'ADN plasmidique de pBIOC21 doublement digéré par HindIII et EcoRI, et déphosphorylé par l'enzyme phosphatase alcaline d'intestin de veau (New England Biolabs) selon les recommandations du fabricant. La ligation et la transformation ont été réalisées comme décrites au chapitre I. b.
L'ADN plasmidique des clones obtenus, sélectionnés sur 10 µg/ml tétracycline, a été extrait selon la méthode de la lyse alcaline (Stephen et al., 1990) et analysé par digestion enzymatique. Le clone résultant a été appelé pBIOC47. La séquence nucléotidique du cDNA codant pour la chaîne β globine recombinante a été vérifiée par séquençage comme décrit au chapitre I. b. L'ADN plasmidique du vecteur binaire pBIOC47 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

c.3. CONSTRUCTION DU PLASMIDE BINAIRE DE CO-EXPRESSION pBIOC49 CONTENANT LES cDNA CODANT POUR LES GLOBINES, α ET β, POUR UN ADRESSAGE CYTOPLASMIQUE.

Le fragment HindIII-EcoRI portant le cDNA codant pour la chaîne α globine à adressage cytoplasmique a été isolé à partir de pBIOC44 décrit au chapitre II. c. 1., et ligué à l'ADN plasmidique de pBIOC43 doublement digéré par HindIII et EcoRI, et préalablement déphosphorylé par l'enzyme phosphatase alcaline d'intestin de veau (New England Biolabs) selon les recommandations du fabricant. La ligation et la transformation ont été réalisées comme décrite au chapitre I. b. L'ADN plasmidique des clones obtenus, sélectionnés sur 12 µg/ml tétracycline, a été extrait selon la méthode de la lyse alcaline (Stephen et al., 1990) et analysé par digestion enzymatique. Le clone résultant a été appelé pBIOC48.
Le fragment PacI-MluI portant le cDNA codant pour la chaîne β globine à adressage cytoplasmique a été isolé à partir de pBIOC45 décrit au chapitre II. c. 2., et ligué à l'ADN plasmidique de pBIOC48 doublement digéré par PacI et MluI, et déphosphorylé par l'enzyme phosphatase alcaline d'intestin de veau (New England Biolabs) selon les recommandations du fabricant. La ligation et la transformation ont été réalisées comme décrites au chapitre I. b, excepté que la souche E. coli Sure tet⁻ a été utilisée à la place de DH5α. La souche Sure tet⁻ dérive de la souche Sure (Stratagene) rendue sensible à la tétracycline par la perte de l'épisome F'. L'ADN plasmidique des clones obtenus, sélectionnés sur 10 µg/ml tétracycline, a été extrait selon la méthode de la lyse alcaline (Stephen et al., 1990) et analysé par digestion enzymatique. Le clone résultant a été appelé pBIOC49.
La séquence nucléotidique des cDNA codant pour les chaînes α et β de globine recombinante a été vérifiée par séquençage comme décrit au chapitre I. b. L'ADN plasmidique du vecteur binaire pBIOC49 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

**III. CONSTRUCTION DES GENES CHIMERIQUES CODANT POUR LES CHAINES DE GLOBINE, α ET β, PERMETTANT UNE EXPRESSION DE l'HEMOGLOBINE HUMAINE RECOMBINANTE DANS LES MITOCHONDRIES DES FEUILLES DE TABAC.**

Pour obtenir un adressage mitochondrial, la séquence codant pour le peptide transit du précurseur de la sous-unité β de l'ATPase-F1 mitochondriale de Nicotiana plumbaginifolia (ATG GCT TCT CGG AGG CTT CTC GCC TCT CTC CTC CGT CAA TCG GCT CAA CGT GGC GGC GGT CTA ATT TCC CGA TCG TTA GGA AAC TCC ATC CCT AAA TCC GCT TCA CGC GCC TCT TCA CGC GCA TCC CCT AAG GGA TTC CTC TTA AAC CGC GCC GTA CAG TAC) est fusionnée au premier codon de la séquence codant pour d'une part, la chaîne α globine mature (délétion de l'ATG initiateur) et d'autre part, la chaîne β globine mature (délétion de l'ATG initiateur) en maintenant les phases ouvertes de lecture.

La séquence codant pour la sous-unité β de l'ATPase F1 mitochondriale de Nicotiana plumbaginifolia est contenue dans le plasmide pTZ-catp2-1 fourni par Boutry. Ce plasmide correspond au plasmide pTZ18R renfermant l'ADNc (cNP10) comme décrit par Boutry et Chua (1985).

Le peptide transit N-terminal, composé de 54 acides aminés comme défini par Chaumont et al. (1994), a été utilisé lors de la réalisation des constructions.

a. CONSTRUCTION DU PLASMIDE pBIOC50 CONTENANT LE cDNA CODANT POUR L'α GLOBINE POUR UN ADRESSAGE MITOCHONDRIAL.

Pour obtenir un adressage mitochondrial de la chaîne α de globine, la séquence codant pour le peptide transit du précurseur de la sous-unité β de l'ATPase-F1 mitochondriale de Nicotiana plumbaginifolia a été fusionnée au premier codon de la séquence codant pour la chaîne a globine mature en maintenant la phase ouverte de lecture. La séquence de clivage entre les séquences du peptide transit et de la chaîne α globine mature est Tyr - Val.

La séquence codant pour le peptide transit du précurseur de la sous-unité β de l'ATPase-F1 mitochondriale a été amplifiée par PCR sur le plasmide pTZ-catp2-1 à l'aide des 2 oligodésoxynucléotides, WD17 (5' cgcaagcttaaca ATG GCT TCT CGG AGG CTT CTC 3') et WD18 (5' tag aat tC GGC cGG AGA CAG CAC GTA CTG TAC GGC GCG GTT TAA G 3'). L' amorce WD17 apporte le site de restriction HindIII, la séquence aaca favorisant l'initiation de la traduction (Joshi, 1987) et les 7 premiers codons du peptide transit (dont l'ATG initiateur). L'amorce WD18 apporte le site de restriction EcoRI, les 5 premiers codons de la séquence codant la chaîne α globine mature (un site de restriction EagI est créé par mutation silencieuse dans le quatrième codon (CCT modifié en CCg) et les 7 derniers codons de la séquence du peptide transit. L'amplification PCR et le traitement des fragments d'ADN amplifiés ont été réalisées comme décrits dans la troisième étape du chapitre II. a. Puis, ces fragments d'ADN ont été digérés doublement par HindIII et EagI, purifiés par électrophorèse sur gel d'agarose 1,8% et par action du kit "Geneclean II" (BIO101) et clonés aux sites HindIII et EagI du plasmide pBIOC44 décrit au chapitre II. a., préalablement purifié par électrophorèse sur gel d'agarose 0,75% et par le kit "Geneclean II". Le plasmide pBIOC44 a été déphosphorylé par l'enzyme phosphatase alcaline d'intestin de veau (New England Biolabs) selon les recommandations du fabricant. La ligation et la transformation ont été réalisées comme décrites au chapitre I. b. L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC50. La séquence nucléotidique de ce gène chimérique résultant de la fusion traductionnelle entre la séquence codant pour le peptide transit et le cDNA codant pour la chaîne α globine mature a été vérifiée par séquençage comme décrit au chapitre I. b. Le séquençage a révélé deux mutations silencieuses situées au dixième nucléotide (C modifié en A) et au cent-quarante-et-unième (C modifié en G) de la séquence codante pour le peptide transit.

b. CONSTRUCTION DU PLASMIDE pBIOC51 CONTENANT LE cDNA CODANT POUR LA β GLOBINE POUR UN ADRESSAGE MITOCHONDRIAL.

Pour obtenir un adressage mitochondrial de la chaîne β de globine, la séquence codant pour le peptide transit du précurseur de la sous-unité β de l'ATPase-F1 mitochondriale de Nicotiana plumbaginifolia a été fusionnée au premier codon de la séquence codant pour la chaîne β globine mature en maintenant la phase ouverte de lecture. La séquence de clivage entre les séquences du peptide transit et de la chaîne β globine mature est Tyr - Val.

La séquence codant pour le peptide transit du précurseur de la sous-unité β de l'ATPase-F1 mitochondriale a été amplifiée par PCR sur le plasmide pTZ-catp2-1 à l'aide des 2 oligodésoxynucléotides, WD19 (5' gtcattaattaaca ATG GCT TCT CGG AGG CTT CTC GCC TCT C 3') et WD20 (5' aatgagct C GGC cGA CTT CTC CTC AGG AGT CAG GTG CAC GTA CTG TAC GGC GCG GTT TAA G 3'). L'amorce WD19 apporte le site de restriction PacI, la séquence aaca favorisant l'initiation de la traduction (Joshi, 1987) et précédant les 9 premiers codons du peptide transit (dont l'ATG initiateur). L'amorce WD20 apporte le site de restriction SacI, les 10 premiers codons de la séquence codant la chaîne β globine mature (un site de restriction EagI est créé par mutation silencieuse dans le neuvième codon (TCT modifié en TCg)) et les 7 derniers codons de la séquence du peptide transit. L'amplification PCR et le traitement des fragments d'ADN amplifiés ont été réalisées comme décrits dans la troisième étape du chapitre II. a. Puis, ces fragments d'ADN ont été digérés doublement par PacI et EagI, purifiés par électrophorèse sur gel d'agarose 1,8% et par action du kit "Geneclean II" (BI0101) et clonés aux sites PacI et EagI du plasmide pBIOC45 décrit au chapitre II. b., préalablement purifié par électrophorèse sur gel d'agarose 0,75% et par le kit "Geneclean II". Le plasmide pBIOC45 a été déphosphorylé par l'enzyme phosphatase alcaline d'intestin de veau (New England Biolabs) selon les recommandations du fabricant. La ligation et la transformation ont été réalisées comme décrites au chapitre I. b. L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC51. La séquence nucléotidique de ce gène chimérique résultant de la fusion traductionnelle entre la séquence codant pour le peptide transit et le cDNA codant pour la chaîne β globine mature a été vérifiée par séquençage comme décrit au chapitre I. b.

c. CONSTRUCTION DU PLASMIDE BINAIRE DE CO-EXPRESSION pBIOC53 CONTENANT LES cDNA CODANT POUR LES GLOBINES, α ET β, POUR UN ADRESSAGE MITOCHONDRIAL.

Le fragment HindIII-EcoRI portant le cDNA codant pour la chaîne α globine à adressage mitochondrial a été isolé à partir de pBIOC50 décrit au chapitre III. a., et ligué à l'ADN plasmidique de pBIOC43 doublement digéré par HindIII et EcoRI, et déphosphorylé par l'enzyme phosphatase alcaline d'intestin de veau (New England Biolabs) selon les recommandations du fabricant. La ligation et la transformation ont été réalisées comme décrite au chapitre I. b. L'ADN plasmidique des clones obtenus, sélectionnés sur 10 µg/ml tétracycline, a été extrait selon la méthode de la lyse alcaline (Stephen et al., 1990) et analysé par digestion enzymatique. Le clone résultant a été appelé pBIOC52.

Le fragment PacI-MluI portant le cDNA codant pour la chaîne β globine à adressage mitochondrial a été isolé à partir de pBIOC51 décrit au chapitre III. b., et ligué à l'ADN plasmidique de pBIOC52 doublement digéré par PacI et MluI, et déphosphorylé par l'enzyme phosphatase alcaline d'intestin de veau (New England Biolabs). La ligation et la transformation ont été réalisées comme décrites au chapitre II.c.3 en utilisant la souche E. coli Sure tet-. L'ADN plasmidique des clones obtenus, sélectionnés sur 10 µg/ml tétracycline, a été extrait selon la méthode de la lyse alcaline (Stephen et al., 1990) et analysé par digestion enzymatique. Le clone résultant a été appelé pBIOC53.

La séquence nucléotidique des cDNA codant pour les chaînes α et β de globine recombinante permettant un adressage mitochondrial α été vérifiée par séquençage comme décrit au chapitre I. b. L'ADN plasmidique du vecteur binaire pBIOC53 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

**IV. CONSTRUCTION DES GENES CHIMERIQUES CODANT POUR LES CHAINES DE GLOBINE, α ET β, PERMETTANT UNE EXPRESSION DE l'HEMOGLOBINE HUMAINE RECOMBINANTE DANS LES CHLOROPLASTES DES FEUILLES DE TABAC.**

Pour obtenir un adressage chloroplastique, la séquence codant pour le peptide transit du précurseur de la petite sous-unité de la ribulose 1,5-biphosphate carboxylase de Pisum sativum L. (ATG GCT TCT ATG ATA TCC TCT TCA GCT GTG ACT ACA GTC AGC CGT GCT TCT ACG GTG CAA TCG GCC GCG GTG GCT CCA TTC GGC GGC CTC AAA TCC ATG ACT GGA TTC CCA GTT AAG AAG GTC AAC ACT GAC ATT ACT TCC ATT ACA AGC AAT GGT GGA AGA GTA AAG TGC) est fusionnée au premier codon de la séquence codant pour d'une part, la chaîne α globine mature (délétion de l'ATG initiateur) et d'autre part, la chaîne β globine mature (délétion de l'ATG initiateur) en maintenant les phases ouvertes de lecture.

Ce peptide transit N-terminal, composé de 57 acides aminés comme défini par Anderson et al. (1986), a été isolé à partir du plasmide pJIT117 (Guerineau et al., 1988) et utilisé lors de la réalisation des constructions.

a. CONSTRUCTION DU PLASMIDE pBIOC55 CONTENANT LE cDNA CODANT POUR L'α GLOBINE POUR UN ADRESSAGE CHLOROPLASTIQUE.

Pour obtenir un adressage chloroplastique de la chaîne a de globine, la séquence codant pour le peptide transit du précurseur de la petite sous-unité de la ribulose 1,5-biphosphate carboxylase de Pisum sativum L. a été fusionnée au premier codon de la séquence codant pour la chaîne α globine mature en maintenant la phase ouverte de lecture. La séquence de clivage entre les séquences du peptide transit et de la chaîne α globine mature est Cys - Val.

La séquence du peptide transit du précurseur de la petite sous-unité de la ribulose 1,5-biphosphate carboxylase a été amplifiée par PCR sur le plasmide pJIT117 à l'aide des 2 oligodésoxynucléotides, WD21 (5' cgcaagcttaaca ATG GCT TCT ATG ATA TCC TCT TCA GC 3') et WD22 (5' tag aat tC GGC cGG AGA CAG CAC GCA CTT TAC TCT TCC ACC ATT GC 3'). L'amorce WD21 apporte le site de restriction HindIII, la séquence aaca favorisant l'initiation de la traduction (Joshi, 1987) et les 8 premiers codons du peptide transit (dont l'ATG initiateur). L'amorce WD22 apporte le site de restriction EcoRI, les 5 premiers codons de la séquence codant la chaîne α globine mature (un site de restriction EagI est créé par mutation silencieuse dans le quatrième codon (CCT modifié en CCg)) et les 7 derniers codons de la séquence du peptide transit. L'amplification PCR et le traitement des fragments d'ADN amplifiés ont été réalisés comme décrits dans la troisième étape du chapitre II. a.

Puis, ces fragments d'ADN ont été digérés doublement par HindIII et EcoRI et clonés aux sites HindIII et EcoRI du plasmide pNEB193 commercialisé par New England Biolabs. Le plasmide pNEB193 a été déphosphorylé comme décrit en II. a. La ligation et la transformation ont été réalisées comme décrites au chapitre I. b. L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC54. La séquence nucléotidique de ce gène chimérique résultant de la fusion traductionnelle entre la séquence codant pour le peptide transit et le cDNA codant pour la chaîne α globine mature a été vérifiée par séquençage comme décrit au chapitre I. b.

A partir du plasmide pBIOC54, le fragment HindIII-EagI, portant la séquence codant pour le peptide transit du précurseur de la petite sous-unité de la ribulose 1,5-biphosphate carboxylase et les 4 premiers codons de la chaîne α globine mature, a été isolé par double digestion, HindIII (digestion totale) et EagI (digestion partielle). Ce fragment HindIII-EagI, purifié par électrophorèse sur gel d'agarose 1,8% et par action du kit "Geneclean II" (BIO101), a été cloné aux sites HindIII et EagI du plasmide pBIOC44 déphosphorylé comme décrit au chapitre II. a. La ligation et la transformation ont été réalisées comme décrites au chapitre I. b. L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC55. La séquence nucléotidique de ce gène chimérique résultant de la fusion traductionnelle entre la séquence codant pour le peptide transit et le cDNA codant pour la chaîne α globine mature a été vérifiée par séquençage comme décrit au chapitre I. b.

b. CONSTRUCTION DU PLASMIDE pBIOC57 CONTENANT LE cDNA CODANT POUR LA β GLOBINE POUR UN ADRESSAGE CHLOROPLASTIQUE.

Pour obtenir un adressage chloroplastique de la chaîne β de globine, la séquence codant pour le peptide transit du précurseur de la petite sous-unité de la ribulose 1,5-biphosphate carboxylase de Pisum sativum L. a été fusionnée au premier codon de la séquence codant pour la chaîne β globine mature en maintenant la phase ouverte de lecture. La séquence de clivage entre les séquences du peptide transit et de la chaîne β globine mature est Cys - Val.

La séquence codant pour le peptide transit du précurseur de la petite sous-unité de la ribulose 1,5-biphosphate carboxylase a été amplifiée par PCR sur le plasmide pJIT117 à l'aide des 2 oligodésoxynucléotides, WD23 (5' gtcattaattaaca ATG GCT TCT ATG ATA TCC TCT TCA GCT GTG 3') et WD24 (5' aatgagct C GGC cGA CTT CTC CTC AGG AGT CAG GTG CAC GCA CTT TAC TCT TCC ACC 3'). L'amorce WD23 apporte le site de restriction PacI, la séquence aaca favorisant l'initiation de la traduction (Joshi, 1987) et précédant les 10 premiers codons du peptide transit (dont l'ATG initiateur). L'amorce WD24 apporte le site de restriction SacI, les 10 premiers codons de la séquence codant la chaîne β globine mature (un site de restriction EagI est créé par mutation silencisuse dans le neuvième codon (TCT modifié en TCg)) et les 6 derniers codons de la séquence du peptide transit. L'amplification PCR et le traitement des fragments d'ADN amplifiés ont été réalisés comme décrits dans la troisième étape du chapitre II. a. Puis, ces fragments d'ADN ont été digérés doublement par PacI et SacI, purifiés par électrophorèse sur gel d'agarose 1,8% et par action du kit "Geneclean II" (BIO101) et clonés aux sites PacI et SacI du plasmide pNEB193 commercialisé par New England Biolabs. Le plasmide pNEB193 a été déphosphorylé comme décrit en II. a. La ligation et la transformation ont été réalisées comme décrites au chapitre I. b. L'ADN plasmidique des clones obtenus, sélectionnés sur 100 µg/ml ampicilline, a été extrait selon la méthode de la lyse alcaline (Stephen et al., 1990) et analysé par digestion enzymatique. Le clone résultant a été appelé pBIOC56. La séquence nucléotidique de ce gène chimérique résultant de la fusion traductionnelle entre la séquence codant pour le peptide transit et le cDNA codant pour la chaîne β globine mature a été vérifiée par séquençage comme décrit au chapitre I. b.

A partir du plasmide pBIOC56, le fragment PacI-EagI, portant la séquence du peptide transit du précurseur de la petite sous-unité de la ribulose 1,5-biphosphate carboxylase et les 9 premiers codons de la séquence codant la chaîne β globine mature, a été isolé par double digestion, PacI (digestion totale) et EagI (digestion partielle). Ce fragment PacI-EagI, purifié par électrophorèse sur gel d'agarose 1,8% et par action du kit "Geneclean II" (BIO101), a été cloné aux sites PacI et EagI du plasmide pBIOC45 déphosphorylé comme décrit au chapitre II. a. La ligation et la transformation ont été réalisées comme décrites au chapitre I. b. L'ADN plasmidique des clones obtenus, sélectionnés sur 100 μg/ml ampicilline, a été extrait selon la méthode de la lyse alcaline (Stephen et al., 1990) et analysé par digestion enzymatique. Le clone résultant a été appelé pBIOC57. La séquence nucléotidique de ce gène chimérique résultant de la fusion traductionnelle entre la séquence codant pour le peptide transit et le cDNA codant pour la chaîne β globine mature a été vérifiée par séquençage comme décrit au chapitre I. b.

c. CONSTRUCTION DU PLASMIDE BINAIRE DE CO-EXPRESSION pBIOC59 CONTENANT LES cDNA CODANT POUR LES GLOBINES, α ET β, POUR UN ADRESSAGE CHLOROPLASTIQUE.

Le fragment HindIII-EcoRI portant le cDNA codant pour la chaîne α globine à adressage chloroplastique a été isolé à partir de pBIOC55 décrit au chapitre IV. a., et ligué à l'ADN plasmidique de pBIOC43 doublement digéré par HindIII et EcoRI, et déphosphorylé comme décrit en II. a. La ligation et la transformation ont été réalisées comme décrite au chapitre I. b. L'ADN plasmidique des clones obtenus, sélectionnés sur 10 μg/ml tétracycline, a été extrait selon la méthode de la lyse alcaline (Stephen et al., 1990) et analysé par digestion enzymatique. Le clone résultant a été appelé pBIOC58.

Le fragment PacI-MluI portant le cDNA codant pour la chaîne β globine à adressage chloroplastique a été isolé à partir de pBIOC57 décrit au chapitre IV. b., et ligué à l'ADN plasmidique de pBIOC58 doublement digéré par PacI et MluI, et déphosphorylé comme décrit en II. a. La ligation et la transformation ont été réalisées comme décrites au chapitre II.c.3 en utilisant la souche E. coli Sure tet⁻. L'ADN plasmidique des clones obtenus, sélectionnés sur 10 μg/ml tétracycline, a été extrait selon la méthode de la lyse alcaline (Stephen et al., 1990) et analysé par digestion enzymatique. Le clone résultant a été appelé pBIOC59.

La séquence nucléotidique des cDNA codant pour les chaînes α et β de globine recombinante permettant un adressage chloroplastique a été vérifiée par séquençage comme décrit au chapitre I. b. L'ADN plasmidique du vecteur binaire pBIOC59 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

**V. CONSTRUCTION DES GENES CHIMERIQUES CODANT POUR LES CHAINES DE GLOBINE, a ET β, PERMETTANT UNE EXPRESSION DE l'HEMOGLOBINE HUMAINE RECOMBINANTE POUR LA SECRETION DANS LES FEUILLES DE TABAC.**

Pour obtenir la sécrétion, la séquence codant pour le peptide signal (PS) de la sporamine A des racines tubérisées de la patate douce (Murakami et al., 1986 ; Matsuoka et Nakamura, 1991) (ATG AAA GCC TTC ACA CTC GCT CTC TTC TTA GCT CTT TCC CTC TAT CTC CTG CCC AAT CCA GCC CAT TCC), est fusionnée au premier codon de la séquence codant pour d'une part, la chaîne α globine mature (délétion de l'ATG initiateur) et d'autre part, la chaîne β globine mature (délétion de l'ATG initiateur) en maintenant les phases ouvertes de lecture. Ce peptide signal de 23 acides aminés a été isolé à partir du plasmide pMAT103 (Matuoka et Nakamura, 1991) et utilisé lors de la réalisation des constructions.

a. CONSTRUCTION DU PLASMIDE pBIOC60 CONTENANT LE cDNA CODANT POUR L'α GLOBINE POUR LA SECRETION.

Pour obtenir la sécrétion de la chaîne a de globine, la séquence codant pour le peptide signal de la sporamine A de la patate douce a été fusionnée au premier codon de la chaîne α de globine mature en maintenant la phase ouverte de lecture. La séquence de clivage entre les séquences du peptide signal et de la chaîne α de globine mature est Ser-Val.

La séquence codant pour le peptide signal (PS) de la sporamine A des racines tubérisées de patate douce a été amplifiée par PCR sur le plasmide pMAT103 à l'aide des 2 oligodésoxynucléotides, WD25 (5' cgcaagcttaaca ATG AAA GCC TTC ACA CTC GC 3') et WD26 (5' tagaattc GGC cGG AGA CAG CAC GGA ATG GGC TGG ATT GGG CAG G 3'). L'amorce WD25 apporte le site de restriction HindIII, la séquence aaca favorisant l'initiation de la traduction (Joshi, 1987) et les 6 premiers codons du peptide signal (dont l'ATG initiateur). L'amorce WD26 apporte le site de restriction Ecori, les 5 premiers codons de la séquence codant la chaîne α globine mature (un site de restriction EagI est créé par mutation silencieuse dans le quatrième codon (CCT modifié en CCg)) et les 7 derniers codons de la séquence du peptide signal. L'amplification PCR et le traitement des fragments d'ADN amplifiés ont été réalisées comme décrits dans la troisième étape du chapitre II. a. Puis, ces fragments d'ADN ont été digérés doublement par HindIII et EagI, purifiés par électrophorèse sur gel d'agarose 1,8% et par action du kit "Geneclean II" (BIO101) et clonés aux sites HindIII et EagI du plasmide pBIOC44 déphosphorylé décrit au chapitre II. a. La ligation et la transformation ont été réalisées comme décrites au chapitre I. b. L'ADN plasmidique des clones obtenus, sélectionnés sur 100 μg/

ml ampicilline, a été extrait selon la méthode de la lyse alcaline (Stephen et al., 1990) et analysé par digestion enzymatique. Le clone résultant a été appelé pBIOC60. La séquence nucléotidique de ce gène chimérique résultant de la fusion traductionnelle entre la séquence codant pour le peptide signal et le cDNA codant pour la chaîne α globine mature a été vérifiée par séquençage comme décrit au chapitre I. b.

b. CONSTRUCTION DU PLASMIDE pBIOC61 CONTENANT LE cDNA CODANT POUR LA β GLOBINE POUR LA SECRETION.

Pour obtenir la sécrétion de la chaîne β de globine, la séquence codant pour le peptide signal de la sporamine A de la patate douce a été fusionnée au premier codon de la chaîne β de globine mature en maintenant la phase ouverte de lecture. La séquence de clivage entre les séquences du peptide signal et de la chaîne β de globine mature est Ser-Val.

La séquence codant pour le peptide signal (PS) de la sporamine A des racines tubérisées de patate douce a été amplifiée par PCR sur le plasmide pMAT103 à l'aide des 2 oligodésoxynucléotides, WD27 (5' gtcattaattaaca ATG AAA GCC TTC ACA CTC GC 3') et WD28 (5' aatgagct C GGC cGA CTT CTC CTC AGG AGT CAG GTG CAC GGA ATG GGC TGG ATT GGG CAG G 3'). L'amorce WD27 apporte le site de restriction PacI, la séquence aaca favorisant l'initiation de la traduction (Joshi, 1987) et les 6 premiers codons du peptide signal (dont l'ATG initiateur). L'amorce WD28 apporte le site de restriction SacI, les 10 premiers codons de la séquence codant la chaîne β globine mature (un site EagI est créé par mutation silencieuse dans le neuvième codon (TCT modifié en TCg)) et les 7 derniers codons de la séquence du peptide signal. L'amplification PCR et le traitement des fragments d'ADN amplifiés ont été réalisés comme décrits dans la troisième étape du chapitre II. a. Puis, ces fragments d'ADN ont été digérés doublement par PacI et EagI, purifiés par électrophorèse sur gel d'agarose 1,8% et par action du kit "Geneclean II" (BIO101) et clonés aux sites PacI et EagI du plasmide pBIOC45 déphosphorylé décrit au chapitre II. b. La ligation et la transformation ont été réalisées comme décrites au chapitre I. b. L'ADN plasmidique des clones obtenus, sélectionnés sur 100 μg/ml ampicilline, a été extrait selon la méthode de la lyse alcaline (Stephen et al., 1990) et analysé par digestion enzymatique. Le clone résultant a été appelé pBIOC61. La séquence nucléotidique de ce gène chimérique résultant de la fusion traductionnelle entre la séquence codant pour le peptide signal et le cDNA codant pour la chaîne β globine mature a été vérifiée par séquençage comme décrit au chapitre I. b.

c. CONSTRUCTION DU PLASMIDE BINAIRE DE CO-EXPRESSION pBIOC63 CONTENANT LES CDNA CODANT POUR LES GLOBINES, α ET β, POUR LA SECRETION.

Le fragment HindIII-EcoRI portant le cDNA codant pour la chaîne α globine pour la sécrétion a été isolé à partir de pBIOC60 décrit au chapitre V. a., et ligué à l'ADN plasmidique de pBIOC43 doublement digéré par HindIII et EcoRI, et déphosphorylé comme décrit en II. a. La ligation et la transformation ont été réalisées comme décrites au chapitre II.c.3 en utilisant la souche E. coli Sure tet.. L'ADN plasmidique des clones obtenus, sélectionnés sur 10 μg/ml tétracycline, a été extrait selon la méthode de la lyse alcaline (Stephen et al., 1990) et analysé par digestion enzymatique. Le clone résultant a été appelé pBIOC62.

Le fragment PacI-MluI portant le cDNA codant pour la chaîne β globine pour la sécrétion a été isolé à partir de pBIOC61 décrit au chapitre V. b., et ligué à l'ADN plasmidique de pBIOC62 doublement digéré par PacI et MluI, et déphosphorylé comme décrit en II. a. La ligation et la transformation ont été réalisées comme décrites au chapitre I. b. L'ADN plasmidique des clones obtenus, sélectionnés sur 10 μg/ml tétracycline, a été extrait selon la méthode de la lyse alcaline (Stephen et al., 1990) et analysé par digestion enzymatique. Le clone résultant a été appelé pBIOC63.

La séquence nucléotidique des cDNA codant pour les chaînes α et β de globine pour la sécrétion a été vérifiée par séquençage comme décrit au chapitre I. b. L'ADN plasmidique du vecteur binaire pBIOC63 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

**VI. CONSTRUCTION DES GENES CHIMERIQUES CODANT POUR LES CHAINES DE GLOBINE, α ET β, PERMETTANT UNE EXPRESSION DE l'HEMOGLOBINE HUMAINE RECOMBINANTE DANS LE RETICULUM ENDOPLASMIQUE DES FEUILLES DE TABAC.**

La séquence codant pour le signal KDEL (Lys-Asp-Glu-Leu) placée à l'extrémité C-terminale des chaînes α et β de globine en amont du codon stop combinée à la présence de la séquence codant pour le peptide signal N-terminal (PS) de la sporamine A des racines tubérisées de patate douce permet un adressage dans le réticulum endoplasmique.

## a. CONSTRUCTION DU PLASMIDE pBIOC65 CONTENANT LE cDNA CODANT POUR L'α GLOBINE PERMETTANT LA RETENTION DANS LE RETICULUM ENDOPLASMIQUE.

Pour obtenir une rétention dans le reticulum endoplasmique, la séquence codant pour le signal KDEL (5' aaa gat gag cta 3') a été placée avant le premier codon stop (TGA) de la chaîne α globine mature en maintenant la phase ouverte de lecture.

Le plasmide contenant le cDNA codant pour la chaîne α globine qui renferme la séquence codant pour le signal KDEL placée avant son premier codon stop a été obtenu en suivant les mêmes étapes que pour la fabrication du plasmide pBIOC44 décrit en II. a., excepté que l'amorce WD29 (5' gcgaattc TCA tag ctc atc ttt ACG GTA TTT GGA GGT CAG CAC 3') remplace l'amorce WD14. L'amorce WD29 apporte le site de restriction EcoRI et la séquence KDEL situés respectivement après et avant le codon stop.

Le plasmide résultant obtenu a été appelé pBIOC64. La séquence nucléotidique du gène chimérique entre le cDNA codant pour la chaîne α globine et la séquence codant pour le signal KDEL α été vérifiée par séquençage comme décrit au chapitre I. b.

Puis, le plasmide pBIOC64 a été modifié comme décrit en V. a. par fusion traductionnelle avec le peptide signal de la sporamine A des racines tubérisées de patate douce pour conduire au plasmide pBIOC65 permettant un adressage dans le réticulum endoplasmique. La séquence nucléotidique du gène chimérique entre la séquence codant pour le peptide signal, le cDNA codant pour la chaîne α globine mature et la séquence codant pour le signal KDEL a été vérifiée par séquençage comme décrit au chapitre I. b. La séquence de clivage entre les séquences du peptide signal et de la chaîne α globine mature est Ser - Val.

## b. CONSTRUCTION DU PLASMIDE pBIOC67 CONTENANT LE cDNA CODANT POUR LA β GLOBINE PERMETTANT LA RETENTION DANS LE RETICULUM ENDOPLASMIQUE.

Pour obtenir une rétention dans le reticulum endoplasmique, la séquence codant pour le signal KDEL (5' aaa gat gag cta 3') a été placée avant le premier codon stop (TAA) de la chaîne β globine mature en maintenant la phase ouverte de lecture.

Le plasmide contenant le cDNA codant pour la chaîne β globine qui renferme la séquence codant pour le signal KDEL avant son premier codon stop a été obtenu en suivant les mêmes étapes que pour la fabrication du plasmide pBIOC45 décrit en II. b., excepté que l'amorce WD30 (5' aatgagctcgttaacgcgt TTA tag ctc atc ttt GTG ATA CTT GTG GGC CAG GGC 3') remplace l'amorce WD16. L'amorce WD30 apporte les sites de restriction MluI, HpaI et SacI, et la séquence KDEL placés respectivement après et avant le codon stop.

Le plasmide résultant obtenu a été appelé pBIOC66. La séquence nucléotidique du gène chimérique entre le cDNA codant pour la chaîne β globine et la séquence codant pour le signal KDEL a été vérifiée par séquençage comme décrit au chapitre I. b.

Puis, le plasmide pBIOC66 a été modifié comme décrit en V. b. par fusion traductionnelle avec le peptide signal de la sporamine A des racines tubérisées de patate douce pour conduire au plasmide pBIOC67 permettant un adressage dans le réticulum endoplasmique. La séquence nucléotidique du gène chimérique entre la séquence codant pour le peptide signal, le cDNA codant pour la chaîne β globine mature et la séquence codant pour le signal KDEL a été vérifiée par séquençage comme décrit au chapitre I. b. La séquence de clivage entre les séquences du peptide signal et de la chaîne β globine mature est Ser - Val.

## c. CONSTRUCTION DU PLASMIDE BINAIRE DE CO-EXPRESSION pBIOC69 CONTENANT LES cDNA CODANT POUR LES GLOBINES, α ET β, PERMETTANT LA RETENTION DANS LE RETICULUM ENDOPLASMIQUE.

Le fragment HindIII-EcoRI portant le cDNA codant pour la chaîne α globine permettant la rétention dans le réticulum endoplasmique a été isolé à partir de pBIOC65 décrit au chapitre VI. a., et ligué à l'ADN plasmidique de pBIOC43 doublement digéré par HindIII et EcoRI, et déphosphorylé comme décrit en II. a. La ligation et la transformation ont été réalisées comme décrite au chapitre I. b. L'ADN plasmidique des clones obtenus, sélectionnés sur 10 µg/ml tétracycline, a été extrait selon la méthode de la lyse alcaline (Stephen et al., 1990) et analysé par digestion enzymatique. Le clone résultant a été appelé pBIOC68.

Le fragment PacI-MluI portant le cDNA codant pour la chaîne β globine permettant la rétention dans le réticulum endoplasmique a été isolé à partir de pBIOC67 décrit au chapitre VI. b., et ligué à l'ADN plasmidique de pBIOC68 doublement digéré par PacI et MluI, et déphosphorylé comme décrit en II.a. La ligation et la transformation ont été réalisées comme décrites au chapitre II.c.3 en utilisant la souche E. coli Sure tet⁻. L'ADN plasmidique des clones obtenus, sélectionnés sur 10 µg/ml tétracycline, a été extrait selon la méthode de la lyse alcaline (Stephen et al., 1990) et analysé par digestion enzymatique. Le clone résultant a été appelé pBIOC69.

La séquence nucléotidique des cDNA codant pour les chaînes α et β de globine permettant leur rétention dans le réticulum endoplasmique a été vérifiée par séquençage comme décrit au chapitre I. b. L'ADN plasmidique du vecteur binaire pBIOC69 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

**VII. CONSTRUCTION DES GENES CHIMERIQUES CODANT POUR LES CHAINES DE GLOBINE, α ET β, PERMETTANT UNE EXPRESSION DE I'HEMOGLOHINE HUMAINE RECOMBINANTE DANS LES VACUOLES DES FEUILLES DE TABAC.**

Pour permettre l'adressage vacuolaire, la séquence codant pour le prépropeptide (PPS) de la sporamine A des racines tubérisées de la patate douce (Murakami et al., 1986 ; Matsuoka et Nakamura, 1991), qui correspond au peptide signal suivi de la séquence N-terminale d'adressage vacuolaire (ATG AAA GCC TTC ACA CTC GCT CTC TTC TTA GCT CTT TCC CTC TAT CTC CTG CCC AAT CCA GCC CAT TCC AGG TTC AAT CCC ATC CGC CTC CCC ACC ACA CAC GAA CCC GCC), est fusionnée au premier codon de la séquence codant pour d'une part, la chaîne α globine mature (délétion de l'ATG initiateur) et d'autre part, la chaîne β globine mature (délétion de l'ATG initiateur) en maintenant les phases ouvertes de lecture. Ce prépropeptide de 37 acides aminés a été isolé à partir du plasmide pMAT103 (Matuoka et Nakamura, 1991) et utilisé lors de la réalisation des constructions.

Pour obtenir un adressage vacuolaire de la chaîne α de globine, la séquence codant pour le prépropeptide de la sporamine A de la patate douce a été fusionnée au premier codon de la chaîne α de globine mature en maintenant la phase ouverte de lecture. La séquence de clivage entre les séquences du peptide signal et de la chaîne α de globine mature est Ala-Val.

a. CONSTRUCTION DU PLASMIDE pBIOC70 CONTENANT LE cDNA CODANT POUR L'α GLOBINE PERMETTANT UN ADRESSAGE VACUOLAIRE.

La séquence codant pour le prépropeptide N-terminal (PPS) de la sporamine A des racines tubérisées de patate douce a été amplifiée par PCR sur le plasmide pMAT103 à l'aide des 2 oligodésoxynucléotides, WD25 (5' cgcaagcttaaca ATG AAA GCC TTC ACA CTC GC 3') décrit en V. a. et WD31 (5' tagaattc GGC cGG AGA CAG CAC GGC GGG TTC GTG TGT GGT TG 3'). L'amorce WD31 apporte le site de restriction EcoRI, les 5 premiers codons de la séquence codant la chaîne α globine mature (un site EagI est créé par mutation silencieuse dans le quatrième codon (CCT modifié en CCg)) et les 6 derniers codons de la séquence du prépropeptide N-terminal. L'amplification PCR et le traitement des fragments d'ADN amplifiés ont été réalisées comme décrits dans la troisième étape du chapitre II. a. Puis, ces fragments d'ADN ont été digérés doublement par HindIII et EagI, purifiés par électrophorèse sur gel d'agarose 1,8% et par action du kit "Geneclean II" (BIO101) et clonés aux sites HindIII et EagI du plasmide pBIOC44 déphosphorylé décrit au chapitre II. a. La ligation et la transformation ont été réalisées comme décrites au chapitre I. b. L'ADN plasmidique des clones obtenus, sélectionnés sur 100 μg/ml ampicilline, a été extrait selon la méthode de la lyse alcaline (Stephen et al., 1990) et analysé par digestion enzymatique. Le clone résultant a été appelé pBIOC70. La séquence nucléotidique du gène chimérique entre la séquence codant pour le prépropeptide et le cDNA codant pour la chaîne α globine mature a été vérifiée par séquençage comme décrit au chapitre I. b.

b. CONSTRUCTION DU PLASMIDE pBIOC71 CONTENANT LE cDNA CODANT POUR LA β GLOBINE PERMETTANT UN ADRESSAGE VACUOLAIRE.

Pour obtenir un adressage vacuolaire de la chaîne β de globine, la séquence codant pour le prépropeptide de la sporamine A de la patate douce a été fusionnée au premier codon de la chaîne β de globine mature en maintenant la phase ouverte de lecture. La séquence de clivage entre les séquences du peptide signal et de la chaîne β de globine mature est Ala-Val.

La séquence codant pour le prépropeptide N-terminal (PPS) de la sporamine A des racines tubérisées de patate douce a été amplifiée par PCR sur le plasmide pMAT103 à l'aide des 2 oligodésoxynucléotides, WD27 (5' gtcattaattaaca ATG AAA GCC TTC ACA CTC GC 3') décrit en V. b. et WD32 (5' aatgagct C GGC cGA CTT CTC CTC AGG AGT CAG GTG CAC GGC GGG TTC GTG TGT GGT TG 3'). L'amorce WD32 apporte le site de restriction SacI, les 10 premiers codons de la séquence codant la chaîne β globine mature (un site de restriction EagI est créé par mutation silencieuse dans le neuvième codon (TCT modifié en TCg)) et les 6 derniers codons de la séquence du prépropeptide N-terminal. L'amplification PCR et le traitement des fragments d'ADN amplifiés ont été réalisés comme décrits dans la troisième étape du chapitre II. a. Puis, ces fragments d'ADN ont été digérés doublement par PacI et EagI, purifiés par électrophorèse sur gel d'agarose 1,8% et par action du kit "Geneclean II" (BIO101) et clonés aux sites PacI et EagI du plasmide pBIOC45 déphosphorylé décrit au chapitre II. b. La ligation et la transformation ont été réalisées comme décrites au chapitre I. b. L'ADN plasmidique des clones obtenus, sélectionnés sur 100 μg/ml ampi-

cilline, a été extrait selon la méthode de la lyse alcaline (Stephen et al., 1990) et analysé par digestion enzymatique. Le clone résultant a été appelé pBIOC71. La séquence nucléotidique du gène chimérique entre la séquence codant pour le prépropeptide et le cDNA codant pour la chaîne β globine mature a été vérifiée par séquençage comme décrit au chapitre I. b.

c. CONSTRUCTION DU PLASMIDE BINAIRE DE CO-EXPRESSION pBIOC73 CONTENANT LES cDNA CODANT POUR LES GLOBINES, α ET β, PERMETTANT UN ADRESSAGE VACUOLAIRE.

Le fragment HindIII-EcoRI portant le cDNA codant pour la chaîne α globine à adressage vacuolaire a été isolé à partir de pBIOC70 décrit au chapitre VII. a., et ligué à l'ADN plasmidique de pBIOC43 doublement digéré par HindIII et EcoRI, et déphosphorylé comme décrit en II.a. La ligation et la transformation ont été réalisées comme décrite au chapitre I. b. L'ADN plasmidique des clones obtenus, sélectionnés sur 10 μg/ml tétracycline, a été extrait selon la méthode de la lyse alcaline (Stephen et al., 1990) et analysé par digestion enzymatique. Le clone résultant a été appelé pBIOC72.

Le fragment PacI-MluI portant le cDNA codant pour la chaîne β globine à adressage vacuolaire a été isolé à partir de pBIOC71 décrit au chapitre VII. b., et ligué à l'ADN plasmidique de pBIOC72 doublement digéré par PacI et MluI, et déphosphorylé comme décrit en II.a. La ligation et la transformation ont été réalisées comme décrites au chapitre II.c.3 en utilisant la souche E. coli Sure tet⁻. L'ADN plasmidique des clones obtenus, sélectionnés sur 10 μg/ml tétracycline, a été extrait selon la méthode de la lyse alcaline (Stephen et al., 1990) et analysé par digestion enzymatique. Le clone résultant a été appelé pBIOC73.

La séquence nucléotidique des cDNA codant pour les chaînes α et β de globine à adressage vacuolaire a été vérifiée par séquençage comme décrit au chapitre I. b. L'ADN plasmidique du vecteur binaire pBIOC73 a été introduit par transformation directe dans la souche LBA4404 d'Agrobacterium tumefaciens selon le procédé de Holsters et al. (1978). La validité du clone retenu a été vérifiée par digestion enzymatique de l'ADN plasmidique introduit.

**VIII : OBTENTION DE PLANTES DE TABAC TRANSGENIQUES**

Les plantes de tabac utilisées pour les expériences de transformation (Nicotiana tabacum var. PBD6) sont cultivées in vitro sur le milieu de base de Murashige et Skoog (1962) additionné des vitamines de Gamborg et al. (1968, Sigma référence M0404), de saccharose à 20g/L et d'agar (Merck) à 8g/L. Le pH du milieu est ajusté à 5,8 avec une solution de potasse avant autoclavage à 120°C pendant 20 min. Les plantules de tabac sont repiquées par bouture des entre-noeuds tous les 30 jours sur ce milieu de multiplication MS20.

Toutes les cultures in vitro sont réalisées en enceinte climatisée, dans les conditions définies ci-dessous :

- Intensité lumineuse de $30\mu E.m^{-2}.S^{-1}$ ; photopériode de 16h ;
- Thermopériode de 26°C le jour, 24°C la nuit.

La technique de transformation utilisée est dérivée de celle de Horsch et al. (1985).

Une préculture d'Agrobacterium tumefaciens souche LBA4404 contenant les plasmides pBIOC46 ou pBIOC47 ou pBIOC49 ou pBIOC53 ou pBIOC59 est réalisée durant 48h à 28°C sous agitation, dans du milieu LB additionné des antibiotiques adéquats (rifampicine et tétracycline). La préculture est ensuite diluée au 50ème dans le même milieu et cultivée dans les mêmes conditions. Après une nuit, la culture est centrifugée (10 min, 3000 g), les bactéries sont reprises dans un volume équivalent de milieu MS30 (30g/L saccharose) liquide et cette suspension est diluée au 10ème.

Des explants d'environ $1cm^2$ sont découpés à partir des feuilles des plantules décrites ci-dessus. Ils sont ensuite mis au contact de la suspension bactérienne pendant 1h, puis séchés rapidement sur papier filtre et placés sur un milieu de coculture (MS30 solide).

Après 2 jours, les explants sont transférés en boîtes de Pétri sur le milieu de régénération MS30, contenant un agent sélectif, la kanamycine (200mg/L), un bactériostatique, l'augmentin (400mg/L) et les hormones nécessaires à l'induction de bourgeons (BAP, 1mg/L et ANA, 0,1mg/L). Un repiquage des explants est effectué sur le même milieu après 2 semaines de culture. Après 2 nouvelles semaines, les bourgeons sont repiqués en boites de Pétri sur le milieu de développement composé du milieu MS20 additionné de kanamycine et d'augmentin. Après 15 jours, les bourgeons sont repiqués en pots sur le même milieu dont la concentration en kanamycine a été diminuée de moitié. L'enracinement prend environ 20 jours, au terme desquels les plantules peuvent être clonées par bouture d'entre-noeuds in vitro ou sorties en serre.

## IX : EXTRACTION ET PURIFICATION PARTIELLES DE PROTEINES RECOMBINANTES A PARTIR DE FEUILLES DE TABAC

Cinquante grammes de feuilles de tabac (poids frais) transformées sont broyés dans l'azote liquide puis mis à agiter durant 15min à 4°C dans 300ml de tampon tris-HCl 50mM pH 8 additionné d'EDTA 1mM, de β-mercaptoéthanol 1mM et de polyvinylpyrrolidone (PVP, 10g/300ml). Le broyat est filtré sur miracloth puis centrifugé pendant 20min à 4°C à 10000 g. Le surnageant est à nouveau filtré sur miracloth. Les protéines sont alors précipitées durant 12 h à 4°C par une solution de sulfate d'ammonium à saturation. Après centrifugation 20 min à 10000 g, le culot est repris dans du tampon tris-HCl 50mM pH 8 additionné de DTT 1mM et d'EDTA 1mM et dialysé 2 fois 12 heures contre ce même tampon. Après dialyse, le rétentat est centrifugé puis filtré sur miracloth. Un dosage de protéines est également effectué selon la technique de Bradford (1976).

Première étape de purification : Equilibration en tampon phosphate 10mM pH 6,7-EDTA 1 mM par passage sur une résine Sephadex G25 puis dépôt sur une résine échangeuse d'ions (CM cellulose) équilibrée en tampon phosphate 10 mM pH 6,7 EDTA 1 mM. Lavage par 4 volumes de ce même tampon puis élution par un gradient linéaire de $Na_2HPO_4$ 10 mM pH 6,7 EDTA 1 mM à $Na_2HPO_4$ 100 mM pH 6,7 EDTA 1 mM.

Deuxième étape de purification : Equilibration en tampon Tris-HCl 10 mM pH 8,4-EDTA 1 mM par passage sur une résine Sephadex G25 puis dépôt sur une résine échangeuse d'ions DEAE-Sephacel équilibrée en tampon Tris-HCl 10 mM pH 8,4 EDTA 1 mM. Lavage par 4 volumes de ce même tampon puis élution par un tampon $KH_2PO_4$ 20 mM pH 7,4. Les conditions de pH et de force ionique peuvent être modifiées selon la nature du variant d'hémoglobine.

Détection de l'hémoglobine

L'hémoglobine (Hb) se détecte grâce à son chromophore, l'hème, qui lui donne sa couleur caractéristique. A faible concentration et en présence d'autre chromophore ou molécule qui diffuse la lumière, le signal dû à l'Hb peut être masqué. Ce problème peut être surmonté grâce à une technique dynamique permettant de détecter la présence de l'Hb dans un système complexe.

Cette méthode repose sur des spectres différentiels correspondant à une transition entre deux formes de l'Hb et sur les propriétés de photodissociation des ligands comme $O_2$ et CO (Gibson, 1956 ; Mardenet et al., 1994). La probabilité de dissociation est plus forte pour le CO, donc ce ligand est utilisé de préférence. La préparation des échantillons s'effectue en anaérobiose.

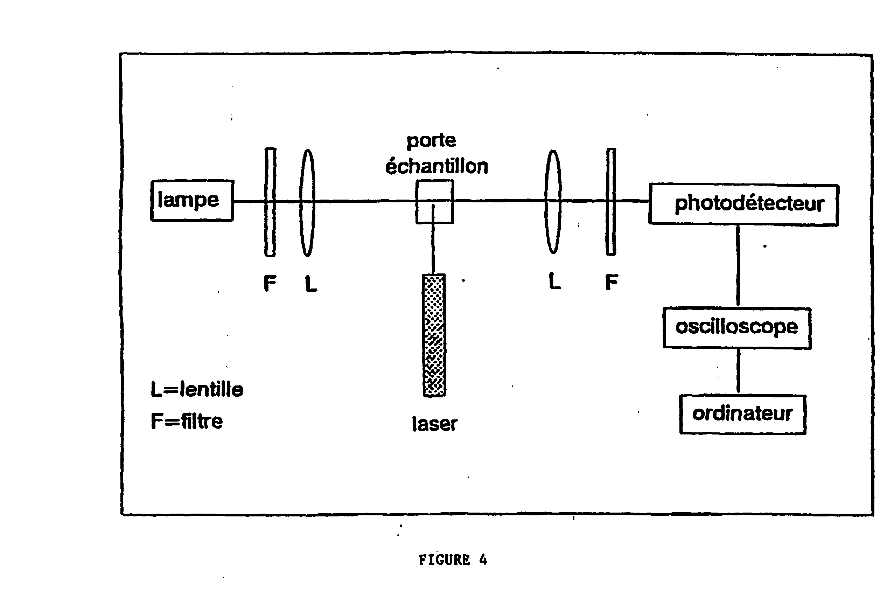

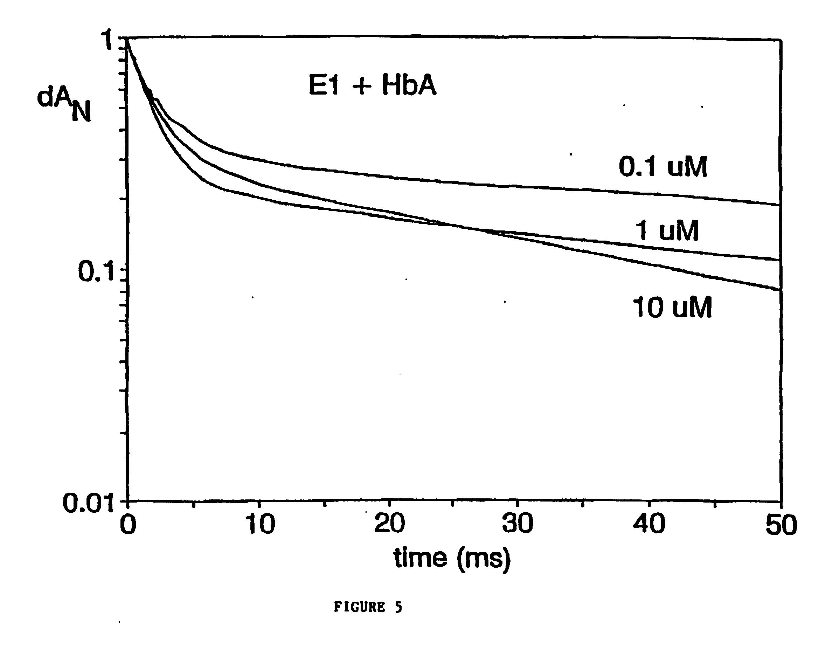

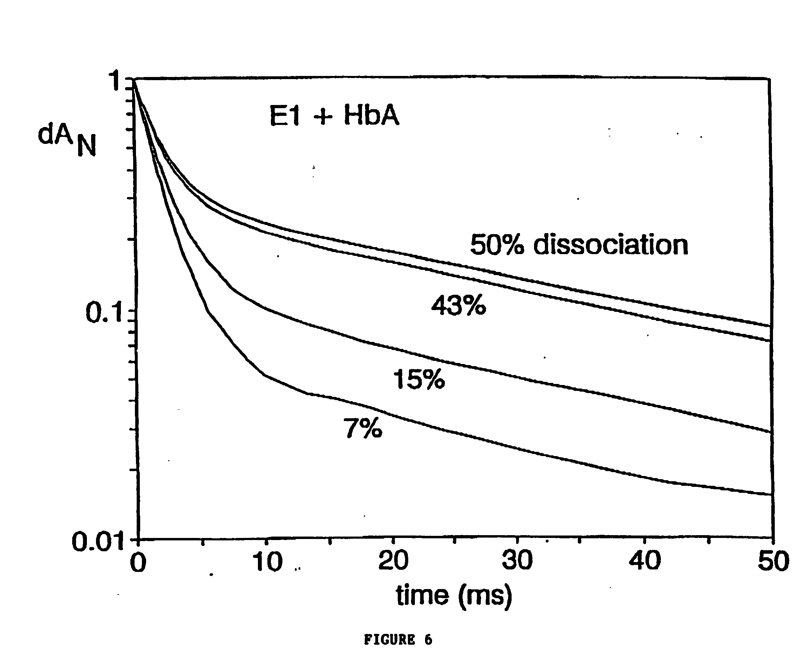

L'équipement expérimental se compose de deux sources de lumières : la première est une source pulsée (laser) qui dissocie les ligands, et la deuxième est une lampe continue qui permet d'observer la recombinaison des ligands, grâce à un changement de l'intensité de lumière transmise (figures 4, 5 et 6). La photodissociation est efficace dans tout le domaine spectral visible ; notre système consiste en un laser YAG dont les impulsions ont une durée de 10 ns à 532 mm. La détection est plus sensible dans la bande de Soret (416 nm) ; nous avons choisi 436 nm proche de l'absorption maximale de la forme désoxy. Les changements d'intensité transmise se produisent tout d'abord dans un temps de l'ordre de la nanoseconde (phase géminée) puis se poursuivent en quelques millisecondes (phase bimoléculaire). Nous nous intéressons particulièrement à cette seconde phase qui reflète les transitions allostériques de l'Hb (figures 5 et 6). Les études cinétiques, rapides et réversibles, permettent d'obtenir de nombreuses données et donc une indication fiable de l'état de l'Hb quant à son fonctionnement normal, physiologique.

La préparation des échantillons est réalisée comme décrite ci-après. Les feuilles de tabac (20 g) sont broyées dans de l'azote liquide, puis le broyat est mélangé à 60 ml de la solution d'extraction (Tris-HCl 25 mM pH7,5, β-mercaptoéthanol 10 mM, EDTA 1 mM). L'homogénat est centrifugé à 10 000g à 4°C pendant 15 minutes. Le surnageant contenant les protéines solubles est prélevé. Le dosage des protéines est effectué selon la technique de Bradford (1976). A 1 ml d'extrait de protéines végétales (1 mg/ml), 32 μl et 3.2 μl d'une solution d'hémoglobine humaine concentrée (3.13 mg/ml) sont ajoutés pour obtenir respectivement des solutions contenant 100%, 10% et 1% d'hémoglobine par rapport aux protéines totales.

Les résultats obtenus sont les suivants :

Les cinétiques des échantillons équilibrés sous 0.1 atm CO pour trois concentrations en Hb : 100%, 10% et 1% des protéines totales présentes dans l'extrait à raison de 1 mg/ml ont été mesurées. Les courbes sont biphasiques, semblables à celles de l'Hb seule, et présentant une vitesse normale (de l'ordre de 1000/s) pour la phase rapide (figure 5). Les cinétiques sont similaires pour les deux concentrations, à l'exception de l'augmentation du bruit (signal) prévisible à faible concentration. Aucun signal n'a été observé pour l'extrait végétal en absence d'Hb, dans les mêmes conditions. Nous pouvons conclure que la cinétique de recombinaison du CO à l'HbA dans un extrait de feuilles de tabac est normale.

**X : EXTRACTION ET PURIFICATION PARTIELLE DE L'BEMOGLOHINE RECOMBINANTE A PARTIR DE GRAINES DE TABAC.**

Dans ce chapitre, on décrit les techniques utilisées, pour la détection par western blot, l'extraction et purification partielle et la mise en évidence de la fonctionnalité de l'hémoglobine recombinante produite dans les graines de tabacs transgéniques (rHb). Ceux-ci sont obtenus par transformation du plasmide de coexpression pBIOC 59 contenant les cDNA codant pour les globines, $\alpha$ et $\beta$, permettant un adressage dans le chloroplaste.

a. DETECTION EN WESTERN BLOT DE L'HEMOGLOBINE RECOMBINANTE ACCUMULEE DANS LES GRAINES DE TABAC.

Soixante-quinze milligrammes de graines de tabac (poids frais) sont broyés dans l'azote liquide puis dans 600 µl de tampon Tris-HCl 25 mM pH 8 glacé additionné d'EDTA 1 mM, de DTT 1 mM et de PMSF 1 mM. Le broyat est transféré dans un tube Eppendorf et centrifugé à 4°C à 10000 g pendant 10 min. Le surnageant est alors concentré par ultrafiltration à l'aide des dispositifs micropure .45 et microcon 10 (Amicon). Le dosage des protéines est effectué selon la technique de Bradford (1976), en utilisant comme étalon l'albumine de sérum de boeuf (fraction V).

Les protéines sont séparées selon leur masse moléculaire apparente par électrophorèse en gel de polyacrylamide en présence de SDS d'après la méthode de Laemmli (Laemmli, 1970) en conditions réductrices. L'appareillage utilisé est le Mini-protean II (Bio-Rad). Le gel est constitué d'un gel de concentration (acrylamide 5 %, bis-acrylamide 0,17 %, Tris-HCl 63 mM pH 6,8, SDS 0,1 %) et d'un gel de séparation (acrylamide 17 %, bis-acrylamide 0,56 %, Tris-HCl 375 mM pH 8,8, SDS 0,1 %). Les échantillons protéiques sont préalablement dilués par 0,25 volume de solution de dépôt (Tris-HCl 200 mM pH 6,8, DTT 400 mM, glycérol 40 %, SDS 8 %, bleu de bromophénol 0,2 %), puis traités à 100°C pendant 5 min et enfin déposés sur le gel. L'électrophorèse est effectuée en tampon Trisglycine-SDS (Tris 25 mM, glycine 250 mM, SDS 1 %) à 25 mA.

Après électrophorèse, les protéines sont transférées sur une membrane de nitrocellulose (BA 85, Schleicher & Schuell) par électrotransfert d'après la technique de Towbin et al. (1979). Le transfert est réalisé à l'aide de l'appareil "mini trans blot module" (Bio-Rad) à 150 V pendant 90 min en présence de la solution de transfert (Tris 25 mM, glycine 192 mM, méthanol 20 %). La membrane est rincée pendant 5 min à température ambiante dans du tampon PBS 1X ($Na_2HPO_4$ 10,4 mM, $KH_2PO_4$ 3,2 mM, NaCl 116 mM), puis séchée.

La présence des chaînes de globine sur les western blot est détectée en utilisant comme anticorps primaire, un immunsérum anti-hémoglobine humaine de lapin (ref : H-4890, Sigma) et comme anticorps secondaire, un anticorps monoclonal anti-IgG de lapin couplé à la phosphatase alcaline (A-8025, Sigma). La révélation est effectuée en utilisant le substrat chromogène [5-bromo-4-chloro-3-indoyl phosphate/nitro blue tetrazolium (BCIP/NBT)].

La membrane est incubée sous agitation pendant 5 min dans une solution tampon TBST (Tris-HCl 10 mM pH 7,5, NaCl 150 mM, Tween 20 0,05 %), puis pendant au moins 30 min dans la même solution additionnée de 5% de lait écrémé en poudre (Régilait). Cette dernière solution est renouvelée, 1/5000 de volume de l'immunsérum anti-hémoglobine est ajouté et la membrane est incubée pendant au moins 2 heures. Elle est rincée 3 fois 5 min par de la solution TBST. L'incubation avec l'anticorps secondaire est effectuée pendant 1 heure avec l'anticorps monoclonal anti-IgG de lapin dilué à 1/10000 dans de la solution TBST. Ensuite, la membrane est de nouveau rincée 3 fois. L'activité phosphatase alcaline est révélée en incubant la membrane dans la solution de révélation (Tris-HCl 100 mM pH 9,5, NaCl 100 mM, $MgCl_2$ 5 mM, BCIP 330 µg/ml, NBT 165 µg/ml). La réaction est arrêtée par rinçage avec de l'eau.

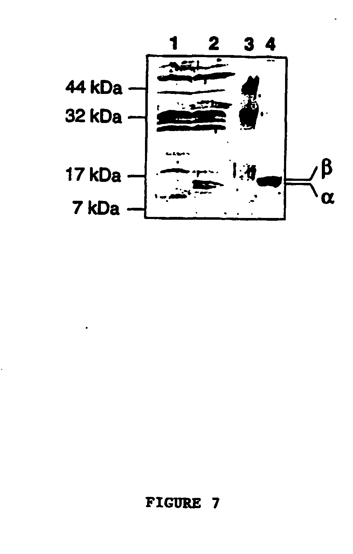

La figure 7 représente l'analyse par western blot de la composition protéique des extraits de graines de tabacs transformés ou non par le plasmide pBIOC59. L'anticorps polyclonal reconnaît les deux chaînes de globine de l'hémoglobine normale adulte (HbA) séparées lors de l'électrophorèse SDS-PAGE. On observe que l'extrait protéique des graines de la plante transgénique T26-22 diffère de celui de la plante témoin par la présence de deux polypeptides dont la masse moléculaire apparente est similaire à celle des chaines de globine de l'HbA et qui sont reconnus par l'anticorps. De plus, ils semblent être représentés de façon équimolaire. On peut donc dire que dans les graines, les transgènes codant pour les protéines de fusion peptide transit-globines $\alpha$ et peptide transit-globine $\beta$ sont exprimés ; le clivage du peptide transit serait correctement effectué, de sorte que s'accumulent les globines $\alpha$ et $\beta$. Dans les graines de 11 plantes, sur les 20 tabacs transformés indépendamment par le plasmide pBIOC59, la présence des globines est détecté. Exprimé en équivalent d'HbA, le taux maximun d'environ 0,05% de rHb par rapport aux protéines totales solubles extraites est observé pour la plante T26-22. Cela a pu être estimé par analyse comparative en western de gammes de concentration d'HbA dans l'extrait protéique de graines de plante témoin.

b. EXTRACTION ET PURIFICATION PARTIELLE DE L'HEMOGLOBINE RECOMBINANTE A PARTIR DE GRAINES DE TABAC.

La purification partielle a été effectuée à partir d'un mélange des graines de tabacs transgéniques transformés par le plasmide pBIOC59 et exprimant la rHb.

Quinze grammes de graines de tabac (poids frais) sont broyés dans l'azote liquide puis dans 100 ml de tampon Tris-HCl 25mM pH 8 glacé additionné d'EDTA 1 mM, de DTT 1 mM et de PMSF 1 mM. Le broyat est filtré sur miracloth®, puis le filtrat est centrifugé à 4°C à 10000 g pendant 10 min. Le surnageant est d'abord saturé en monoxyde de carbone (CO), puis filtré avec un filtre 0,22 μm et enfin concentré par ultrafiltration à l'aide de dispositifs centriprep 10 (Amicon). Le concentrat est saturé en CO. Deux étapes chromatographiques successives sont réalisées (4°C) en suivant les absorbances à 280 nm (protéines) et 415 nm (hémoprotéines). (i) Le concentrat est préalablement filtré avec un filtre 0,22 μm, puis déposé sur colonne Sephacryl-S100 (Pharmacia) (2,1 cm x 90 cm) équilibré avec le tampon D ($Na_2HPO_4$ 9,12 mM, $NaH_2PO_4$ 20,88 mM, DTT 1 mM, EDTA 1 mM, pH 6,5). La fraction contenant la rHb est collectée, filtrée à travers un filtre 0,22 μm, puis saturée en CO et enfin concentrée comme précédemment. Soixante-cinq pour cent des protéines sont éliminées à cette étape. (ii) Ce concentrat est déposé sur la deuxième colonne, une S-sepharose fast-flow (Pharmacia) (1,1 cm x 10 cm) équilibrée avec du tampon D. Après lavage par 8 volumes de tampon D, un gradient de force ionique est appliqué (tampon D à tampon D contenant 500 mM NaCl). L'hémoglobine est éluée en un pic. Les fractions contenant ce pic sont rassemblées et les protéines sont concentrées comme cela est décrit plus haut. Avant et après concentration les échantillons sont saturés en CO. Ce concentrat constitue la fraction enrichie en rHb appelée FE-rHb. Il ne reste plus dans cette fraction que 3 % des protéines de l'extrait. Pour disposer d'un témoin pour les analyses ultérieures, ce schéma de purification a été appliqué dans les mêmes conditions à un extrait obtenu à partir de 15 g de graines de tabacs n'exprimant pas la rHb, conduisant à l'obtention de la fraction appelée FE-Témoin.

La présence des globines α et β dans ces fractions a été recherchée en utilisant la technique de western blot dans les conditions décrites au paragraphe X.a. La fraction FE-rHb contient bien de la rHb, ces deux polypeptides étant mis en évidence (figure 8).

c. MISE EN EVIDENCE DE LA FONCTIONNALITE DE L'HEMOGLOBINE RECOMBINANTE PAR PHOTOLYSE ECLAIR.

La mise en évidence de la fonctionnalité a été réalisée à partir de la fraction enrichie en rHb appelée FE-rHb, en utilisant comme témoin la fraction équivalente témoin FE-Témoin et de l'HbA.

Les expériences de contrôle où l'on a ajouté de l'HbA à l'extrait de plantes, ont montré des cinétiques de recombinaison biphasiques et des variations de la fraction lente selon l'énergie de l'éclair de lumière laser. Ces résultats démontrent que la fonction de l'HbA n'est pas altérée par les conditions de solvant utilisées.

Après photodissociation des ligands de l'Hb, la recombinaison bimoléculaire a lieu dans une échelle de temps de μs-ms (vitesse k-on). Bien que le ligand physiologique naturel soit l'oxygène, les études décrites ont été faites avec du monoxyde de carbone (CO) qui donne un signal de photodissociation très supérieur à celui obtenu avec l'$O_2$ car le rendement est plus élevé. De même la différence des vitesses de recombinaison pour les deux conformations de l'Hb (R et T correspondant à des tétramères avec et sans ligand) est également plus élevée. Expérimentalement les échantillons sont équilibrés sous 0,1 atm CO qui donne les meilleures conditions d'observation des deux phases. Comme la réaction est réversible, on peut répéter la photodissociation (γ) du même échantillon pour accumuler plusieurs courbes ce qui améliore beaucoup le rapport signal/bruit.

$$\gamma$$

$$HbCO \rightarrow Hb + CO$$

$$k\text{-on -CO}$$

L'observation d'une variation de l'amplitude de la recombinaison lente en fonction de la fraction de dissociation (par modification de l'énergie laser), démontre la présence d'une hémoglobine fonctionnelle.

Les plantes transgéniques recoivent l'information génétique uniquement pour la synthèse de globine et non pour l'hème. En conséquence si l'Hb fonctionnelle (globines + hème) est exprimée dans les plantes, c'est qu'elle a capté l'hème in situ. D'autres hémoprotéines présentes dans les plantes peuvent donner un signal optique après photolyse éclair. Ces hémoprotéines ne donneront pas de signal si le fer héminique est sous forme ferrique qui ne lie pas les ligands CO et $O_2$. Le CO et l'$O_2$ ne se lient de façon réversible que si l'atome de fer est sous forme ferreuse. Il est par conséquent important de démontrer l'existence de processus cinétiques pour les deux phases et la variation des contributions relatives des deux phases due à des facteurs connus pour influencer la fonction de l'hémoglobine.

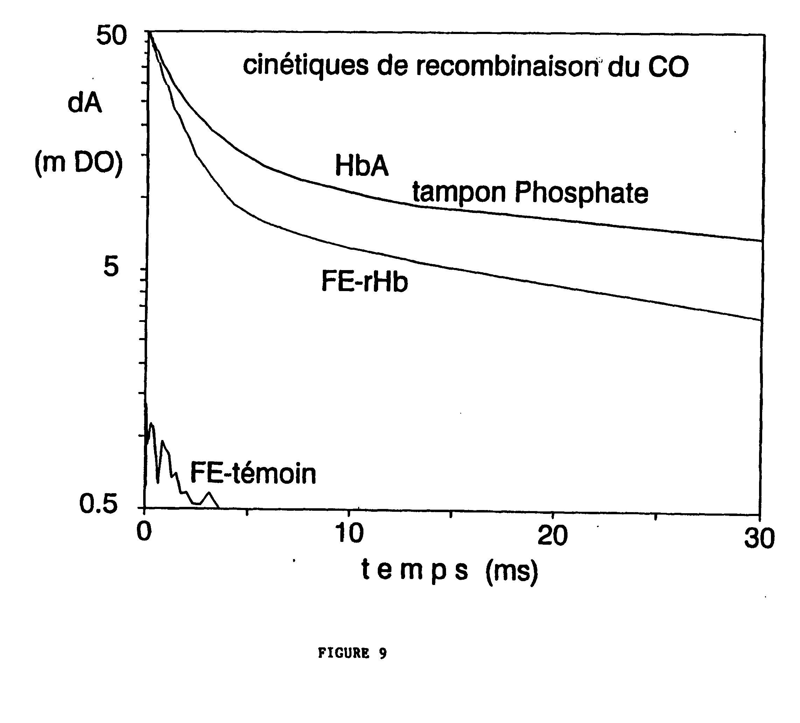

L'échantillon enrichi FE-rHb montre un signal de photodissociation du CO de 48 mDO (densité optique) et permet de réaliser certaines expériences à différents niveaux de dissociation ; ces expériences sont réalisées en l'absence de dithionite de sodium pour éviter toute contribution parasite due à la présence d'hémoproteines. La même expérience, réalisée avec la fraction FE-Témoin a montré un signal de 1 mDO (figure 9).

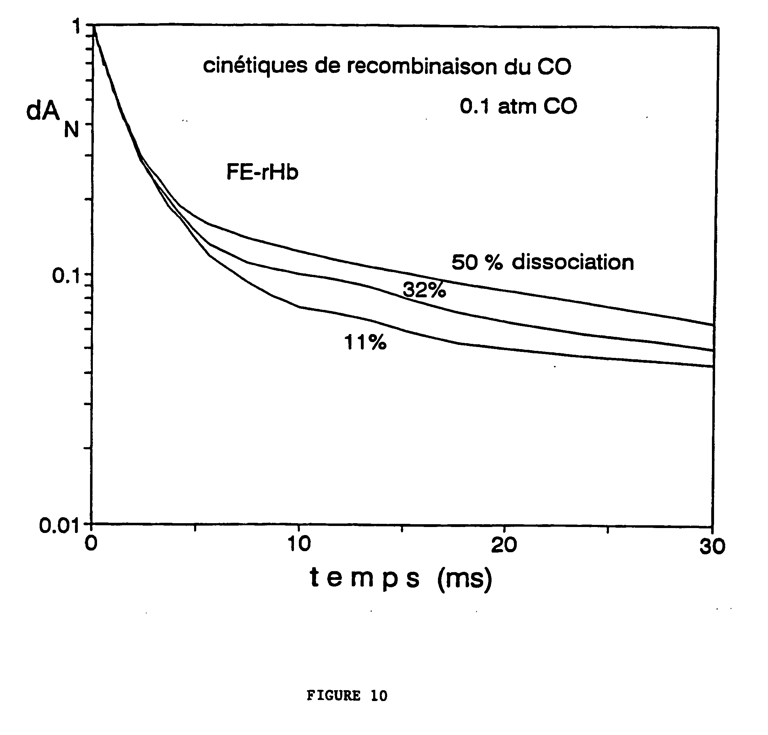

Les résultats enregistrés à différents niveaux d'énergie de lumière laser sont montrés dans la Figure 10. Les courbes sont similaires à celle de l'HbA et montre l'existence d'une propriété caractéristique de l'hémoglobine à savoir la plus faible fraction de vitesse lente quand l'intensité de la lumière est diminuée de façon à obtenir une dissociation plus faible.

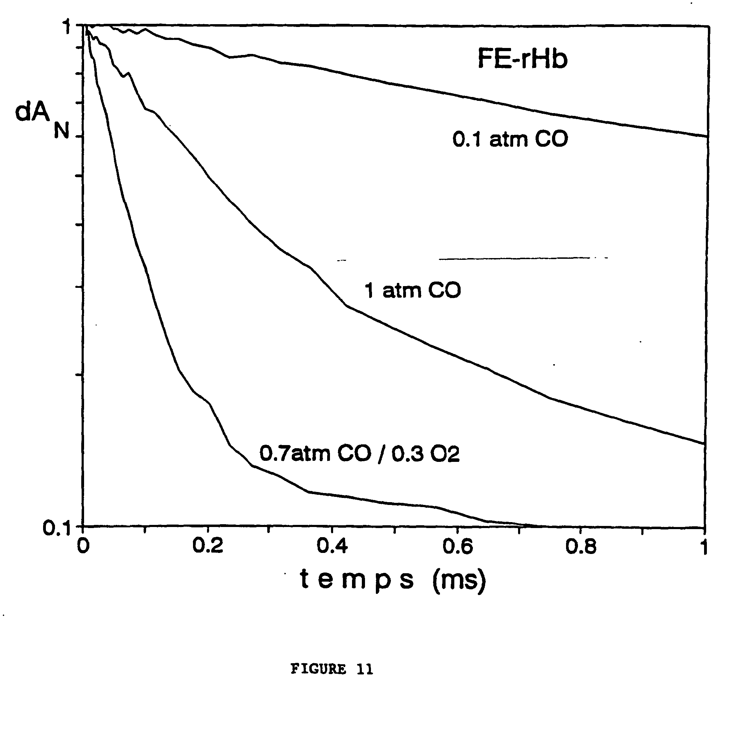

L'échantillon a été ensuite équilibré sous une atmosphère de CO. Comme attendu, les cinétiques de recombinaison sont désormais plus rapides. Pour l'hémoglobine en solution, la fraction lente est habituellement plus basse à fortes concentration de CO puisqu'il y a moins de temps disponible pour faire la transition R->T après dissociation. L'échantillon FE-rHb ne présente pas cet effet (figure 11).

Une autre méthode peut être utilisée pour étudier les vitesses d'association et de dissociation de l'oxygène. Le principe de cette méthode repose sur le fait suivant : bien que le CO ait une affinité environ 200 fois plus élevée que celle de l'oxygène, la vitesse d'association du CO à l'Hb ligandée (R state) est environ 10 fois plus faible que pour l'oxygène. Un échantillon équilibré avec un mélange égal de CO et d'$O_2$ sera essentiellement sous forme de HbCO. On peut alors photodissocier le CO (avec un fort rendement) ce qui permet l'étude de la recombinaison de l'$O_2$. Une phase terminale lente de l'ordre de 1s due au remplacement de l'oxygène par le CO fournit des informations sur la vitesse de dissociation (k-off). Seul l'échantillon FE-rHb révèle un signal de liaison de l'oxygène (figure 11).

Les études de la fraction FE-rHb par photolyse éclair ont montré :

- une recombinaison du CO biphasique avec des vitesses rapide et lente similaires à celles observées dans l'Hb A tétramèrique ;
- une diminution de la fraction lente à énergie faible du laser comme pour l'Hb A ;
- une augmentation de la vitesse pour des concentrations de CO plus élevées comme pour l'Hb normale ;
- une liaison réversible de l'oxygène avec des vitesses on et off similaires à celles de l'Hb A normale ;

Nous concluons que l'hémoglobine recombinante produite dans les graines de tabac possède les propriétés de l'Hb A tétramèrique dans tous les tests fonctionnels réalisés.

**XI : CONSTRUCTION DE GENES CHIMERIQUES CODANT POUR LES CHAINES $\alpha$ ET $\beta$ DE L'HEMOGLOBINE HUMAINE ET PERMETTANT UNE EXPRESSION DANS LES SEMENCES DE MAIS.**

**CONSTRUCTION DES PLASMIDES CONTENANT UNE DES CHAINES $\alpha$ OU $\beta$ DE L'HEMOGLOBINE HUMAINE ET PERMETTANT UNE EXPRESSION CONSTITUTIVE OU UNE EXPRESSION DANS L'ALBUMEN DANS LES SEMENCES DE MAIS.**

L'expression constitutive ou albumen spécifique dans les semences de maïs des séquences des chaînes $\alpha$ et $\beta$ de l'hémoglobine humaine a nécessité les séquences régulatrices suivantes :

un des trois promoteurs permettant une expression constitutive :

- promoteur actine de riz suivi de l'intron actine de riz (pAR-IAR) contenu dans le plasmide pActI-F4 décrit par McElroy et al. (1991) ;
- promoteur constitutif double 35S (pd35S) du CaMV (virus de la mosaïque du chou-fleur). Il correspond à une duplication des séquences activant la transcription situées en amont de l'élément TATA du promoteur 35S naturel (Kay et al., 1987);
- le promoteur du gène de $\gamma$zéine de maïs (p$\gamma$zéine) contenu dans le plasmide p$\gamma$63 (Reina et al., 1990). Le plasmide p$\gamma$63 résulte du clonage de p$\gamma$zéine aux sites HindIII et XbaI d'un plasmide pUC18 renfermant, entre ses sites HindIII et EcoRI, la cassette d'expression "p35S-gus-tNOS" de pBI221 commercialisé par Clontech. Il permet une expression dans l'albumen des semences de maïs. Combiné à l'intron actine de riz, ce promoteur confère une expression est de type constitutive ;

un des deux terminateurs :

- la séquence terminatrice de transcription, terminateur polyA 35S, qui correspond à la région en 3' non codante de la séquence du virus à ADN bicaténaire circulaire de la mosaïque du chou-fleur produisant le transcrit 35S (Franck et al., 1980) ;
- la séquence terminatrice de transcription, terminateur polyA NOS, qui correspond à la région en 3' non codante du gène de la nopaline synthase du plasmide Ti d'Agrobacterium tumefaciens souche à nopaline (Depicker et al., 1982).

Le type de vecteur utilisé dérive de pBSIISK+ (Stratagene). Chaque vecteur comporte une cassette d'expression, à savoir un des promoteurs, une des chaînes α ou β de l'hémoglobine humaine et un des terminateurs. Des vecteurs comportant les deux cassettes d'expression de chacune des chaînes α et β de l'hémoglobine humaine ont également été construits. Les clonages ont été réalisés selon les procédés usuels.

Références bibliographiques :

- Benesch & Kwong. Hemoglobin 1994, 18, 185-192.
- Birnboim & Doly. Nucleic Acids Res. 1979, 7, 1513-
- Boutry & Chua. EMBO J. 1985, 4, 2159-2165.
- Bradford. Anal. Biochem. 1976, 72, 248-254.
- Carrer et al. Mol. Gen. Genet. 1993, 241, 49-56.
- Chaumont et al. Plant Mol. Biol. 1994, 24, 631-641.
- Gamborg et al. Exp. Cell Res. 1968, 50, 151-158.
- Guerineau et al. Nucleic Acid Res. 1988, 16 11380.
- Gibson. J. Physiol. 1956, 134, 123.
- Edelbaum. J. Interferon Res. 1992, 12, 449-453.
- Hanahan. J. MoL Biol. 1983, 166, 557-
- Hanahan. In "DNA cloning volume I, a practical approach" (Ed: Glover D.M.) IRL Press, 1985, pp 109-135.
- Hiatt & Ma. FEBS Let. 1992, 307, 71-75.
- Hoffman et al. Proc. Natl. Acad. Sci. USA 1990, 87 : 8521-8525.
- Horsch et al. Science 1985, 227, 1229-1231.
- International Hemoglobin Information Center (1995) Hemoglobin, 19, 37-124.
- Jessen et al. Meth Enzymol, 1994, 231, 347-364.
- Joshi. Nucleic Acid Res. 1987, 15, 6643-6653.
- Kister et al. J. Biol. Chem. 1987, 262, 12085-12091.
- Krebbers et al. Plant Physiol. 1988, 87, 859-866.
- Marden et al. Meth Enzymol. 1994, 232 71-86.
- Mason et aL Proc. Natl. Acad. Sci. USA 1992, 89, 11745-11749.
- Moloney. Int. Meeting of Production of Recombinant Proteins in Plants, Leicester 1994, page 36-38
- Murashige & Skoog. Physiol. Plantarum 1962, 15, 473-497.
- Nagai & Thogersen. Meth. Enzymol. 1987,153, 461-481.
- Nagai et al. Proc. Natl. Acad. Sci. USA 1985, 82, 7252-7255.
- Peratz. Nature 1970, 228, 726-739.
- Russel. Int. Meeting of Production of Recombinant Proteins in Plants, Leicester 1994, page 43
- Sambrook et al. Molecular Cloning: A Laboratory Manual. Second Edition. Cold Spring Harbor Laboratory Press, Cold Spring Harbor, NY (1989).
- Sanger et al. Proc. NatL Acad. Sci. USA 1977, 74, 5463-5467
- Stephen et al. Nucleic Acid Res. 1990, 18, 7463-7464.
- Svab et al. Proc. Natl. Acad. Sci. USA 1990, 87, 8526-8530.
- Swanson et al. Bio/Technology 1991, 9, 57-61.
- Symons et al. Bio/Technology 1990, 8, 217-221.
- Vanderkerckhove et al. Bio/Technology 1989, 7, 929-932.
- Wagenbach et aL Biotechnology 1991, 9 : 57-61.
- Wilson et al. Nucleic Acid Res. 1978, 5, 563-581.

Kay R. et al., Science, 1987, <u>236</u> : 1299.

Franck A. et al., Cell, 1980, <u>21</u> : 285.

Depicker A. et al., *J*. Mol. Appl. Genet., 1982, <u>1</u> : 561.

Mc Elroy et al., Mol. Gen. Genet., 1991, <u>231</u> : 150.

Reina et al., N.A.R., 1990, 18 : 6426.

Dumoulin et al., Prot. Sci., 1996, 5 : 114-120.

Feng et al., J. Mol. Evol., 1985, 21 : 112-115.

Dumoulin et al., Art. Cells Blood Subs. Immob. Biotech., 1994, 22 : 733-738.

Looker et al., Nature, 1992, 356 : 258-260.

Laemmli, Nature, 1970, 227 : 680-685.

Towbin et al., Proc. Natl. Acad. Sci. USA, 1979, 76 : 4350-4354.

## Revendications

1. Procédé de production de protéines héminiques comprenant les étapes suivantes :

   i) introduction, dans des cellules végétales, d'une ou plusieurs molécule(s) d'acides nucléiques dont chacune comporte au moins une séquence codant pour un composant protéique d'une protéine héminique d'origine animale capable de fixer réversiblement l'oxygène, ou pour une variante d'origine animale ou une partie de ce composant protéique, ladite variante ou ladite partie étant capable de fixer reversiblement l'oxygène, et éventuellement une séquence codant pour un agent de sélection ;
   ii) sélection des cellules contenant l'acide nucléique codant pour le composant protéique de la protéine héminique ;
   iii) éventuellement, propagation des cellules transformées, soit en culture, soit par la régénération de plantes entières transgéniques ou chimériques ;
   iv) récupération, et éventuellement purification, d'une protéine héminique comprenant un complexe constitué de la protéine ou des protéines codées par le susdit acide nucléique et au moins un noyau porphyrique à fer, ou une pluralité de ces complexes.

2. Procédé selon la revendication 1 **caractérisé en ce que** la protéine héminique est à chaîne polypeptidique unique, par exemple la myoglobine.

3. Procédé selon la revendication 1 **caractérisé en ce que** la protéine héminique est un hétéro-oligomère, le ou les acide(s) nucléique(s) comportant les séquences codant pour chacune des différentes unités protéiques.

4. Procédé selon la revendication 3, **caractérisé en ce que** la protéine héminique est l'hémoglobine humaine, ou un dérivé de celle-ci, le ou les acide(s) nucléique(s) comprenant des séquences codant pour l'$\alpha$- et $\beta$-globine, ou pour des variantes de l'$\alpha$- ou de la $\beta$-globine, les variantes se différenciant de la séquence naturelle par une ou plusieurs substitution(s), délétion(s) ou insertion(s) d'acides aminés.

5. Procédé selon la revendication 4 **caractérisé en ce que** les séquences codant pour les différentes unités protéiques, $\alpha$- et $\beta$-globine, sont comprises au sein de la même molécule d'acide nucléique.

6. Procédé selon la revendication 4 **caractérisé en ce que** les séquences codant pour les différentes unités protéiques, $\alpha$- et $\beta$-globine, sont comprises au sein de molécules d'acide nucléique distinctes.

7. Procédé selon l'une quelconque des revendications 1 à 6, **caractérisé en ce que** l'introduction de la ou des molécule(s) d'acide nucléique s'effectue par transformation du génome nucléaire de la cellule végétale.

8. Procédé selon la revendication 7 **caractérisé en ce que** la séquence codant pour le composant protéique comprend une ou plusieurs séquence(s) codant pour des signaux d'adressage chloroplastiques, ou pour des signaux d'adressage mitochondriaux.

9. Procédé selon la revendication 7 **caractérisé en ce que** la séquence codant pour le composant protéique comprend une ou plusieurs séquence(s) codant pour un peptide signal N-terminal et éventuellement un signal responsable de la rétention de la protéine dans le réticulum endoplasmique, ou un signal d'adressage vacuolaire.

10. Procédé selon l'une quelconque des revendications 1 à 6 **caractérisé en ce que** l'introduction de l'acide nucléique s'effectue par la transformation du génome mitochondrial ou chloroplastique.

11. Procédé selon l'une quelconque des revendications 1 à 9 **caractérisé en ce que** l'acide nucléique comprend, outre la ou les séquence(s) codante(s), des séquences régulatrices de transcription reconnues par les cellules végétales.

12. Procédé selon l'une quelconque des revendications 4 à 11 **caractérisé en ce que** la ou les séquence(s) codante (s) code(nt) pour une molécule hybride composée d'au moins les parties actives de l'α-globine et de la β-globine.

13. Procédé selon l'une quelconque des revendications 1 à 12 **caractérisé en ce qu'**il comprend, entre l'étape de propagation et l'étape de récupération, une étape de détection de protéines héminiques, et en particulier de protéines héminiques dont le noyau porphyrique est constitué de protoporphyrine IX à fer.

14. Procédé selon la revendication 13 **caractérisé en ce que** lors de l'étape de propagation des cellules végétales, de la protoporphyrine IX à fer est additionnée au milieu de culture utilisé pour la croissance des cellules.

15. Protéine héminique ayant la capacité de fixer réversiblement l'oxygène, **caractérisée en ce qu'**elle comporte au moins un noyau porphyrique à fer, d'origine végétale, et un composant protéique comprenant au moins une chaîne polypeptidique, d'origine animale.

16. Protéine selon la revendication 15 **caractérisée en ce que** le noyau porphyrique à fer est la protoporphyrine IX à fer, ou une protoporphyrine se distinguant de la protoporphyrine IX par la nature des chaînes latérales portées par les atomes β des cycles pyrrole.

17. Protéine selon la revendication 16 **caractérisée en ce que** le composant protéique comprend au moins une chaîne polypeptidique α- et/ou β-globine, ou des variantes de celles-ci comportant une ou plusieurs substitution(s), délétion(s) ou insertion(s) d'acides aminés, la protéine héminique étant capable de fixer l'oxygène, de manière réversible.

18. Protéine selon la revendication 17 **caractérisée en ce que** la chaîne d'α- ou β-globine, ou les variantes de celles-ci, comprend en outre un signal d'adressage chloroplastique, un signal d'adressage mitochondrial, ou un peptide signal N-terminal éventuellement en combinaison avec un signal responsable de la rétention de la protéine dans le réticulum endoplasmique, ou un signal d'adressage vacuolaire.

19. Protéine selon la revendication 17, **caractérisée en ce que** chaque chaîne polypeptidique est dépourvue de Méthionine $NH_2$-terminale.

20. Protéine selon l'une quelconque des revendications 17 à 19, **caractérisée en ce que** le composant protéique comprend au moins quatre chaînes polypeptidiques d'α- et/ou de β-globine, ou des variantes de celles-ci, chaque chaîne polypeptidique étant liée à un noyau protoporphyrine à fer.

21. Protéine selon la revendication 20 **caractérisée en ce qu'**elle comprend 2 chaînes α-globines et 2 chaînes β-globines, ou des variantes de celles-ci.

22. Protéine selon l'une quelconque des revendications 15 à 21 **caractérisée en ce qu'**elle fixe l'oxygène avec une affinité comprise entre 7 et 40 mm Hg, de préférence 15 à 20 mm Hg.

23. Acide nucléique comportant :

i) une ou plusieurs séquence(s) codant un composant protéique d'une protéine héminique animale, ladite protéine ayant la capacité de fixer réversiblement l'oxygène, et
ii) des séquences régulatrices de transcription reconnues par une cellule végétale, comprenant un promoteur et des séquences régulatrices de terminaison, et
iii) une ou plusieurs séquence(s) codant un signal d'adressage d'origine végétale.

**24.** Acide nucléique selon la revendication 23 **caractérisé en ce que** les séquences régulatrices comprennent un ou plusieurs promoteur(s) d'origine végétale.

**25.** Acide nucléique selon la revendication -23 ou 24 **caractérisé en ce que** les séquences codant le signal d'adressage codent pour un peptide d'adressage mitochondrial ou chloroplastique dit peptide "transit".

**26.** Acide nucléique selon la revendication 23 ou 24 **caractérisé en ce que** les séquences codant le signal d'adressage codent un peptide signal N-terminal d'origine végétale, éventuellement en combinaison avec une séquence codant un signal de rétention endoplasmique ou un signal d'adressage vacuolaire.

**27.** Acide nucléique selon l'une quelconque des revendications 23 à 26 **caractérisé en ce que** la séquence codante code l'α- ou β-globine humaine, ou une variante de celles-ci se différenciant de la séquence naturelle par une ou plusieurs substitution(s), délétion(s) ou remplacement(s) d'acides aminés, ou une partie de l'α- et/ou β-globine humaine.

**28.** Acide nucléique selon l'une quelconque des revendications 23 à 27 comportant en outre un ou plusieurs intron(s) de préférence d'origine végétale.

**29.** Acide nucléique selon l'une quelconque des revendications 23 à 28 **caractérisé en ce que** la séquence codant pour le composant protéique est un cDNA.

**30.** Vecteur comprenant une ou plusieurs molécule(s) d'acide nucléique selon l'une quelconque des revendications 23 à 29.

**31.** Cellules végétales transformées de manière stable par l'acide nucléique selon l'une quelconque des revendications 23 et 29.

**32.** Cellules végétales capables de produire une ou plusieurs protéine(s) héminique(s) selon l'une quelconque des revendications 15 à 22.

**33.** Cellules végétales selon la revendication 32 **caractérisées en ce qu'**elles comportent un acide nucléique comprenant une ou plusieurs séquence(s) codant pour un composant protéique de ladite protéine héminique en association à une ou plusieurs séquence(s) régulatrice(s) de transcription reconnue(s) par la cellule.

**34.** Cellules végétales selon l'une quelconque des revendications 31 à 33 **caractérisées en ce qu'**il s'agit d'une culture de cellules végétales, par exemple en milieu liquide ou immobilisées, ou une culture de racines.

**35.** Cellules végétales selon l'une quelconque des revendications 31 à 33 **caractérisées en ce qu'**il s'agit de cellules faisant partie d'une plante transformée entière.

**36.** Plante chimérique ou transgénique capable de produire une ou plusieurs protéine(s) héminique(s), par exemple l'hémoglobine ou un dérivé de celle-ci, **caractérisée en ce qu'**elle comporte des cellules selon l'une quelconque des revendications 31 à 33.

**37.** Graines de plante transgénique selon la revendication 36.

**38.** Produit pharmaceutique comprenant une ou plusieurs protéine(s) héminique(s) selon l'une quelconque des revendications 15 à 22 en association avec un excipient acceptable du point de vue physiologique.

**39.** Protéines héminiques selon l'une quelconque des revendications 15 à 22 pour utilisation comme médicament.

**40.** Protéine héminique selon la revendication 39 pour une utilisation en thérapie de conditions nécessitant une amélioration du transport d'oxygène dans le sang.

**41.** Utilisation d'une protéine héminique selon l'une quelconque des revendications 15 à 22 pour la préparation d'un médicament pour le traitement de conditions nécessitant une amélioration du transport d'oxygène dans le sang.

**42.** Utilisation d'une protéine héminique selon l'une quelconque des revendications 15 à 22 dans un produit cosmétique, industriel ou en tant que réactif chimique.

**Patentansprüche**

1. Verfahren zur Herstellung von Hämproteinen, das folgende Phasen umfasst:

   i) Einführung eines oder mehrerer Nukleinsäuremoleküle in Pflanzenzellen, wobei jedes Molekül mindestens eine codierende Sequenz enthält, und zwar für einen proteinartigen Bestandteil eines Hämproteins tierischen Ursprungs, der in der Lage ist, den Sauerstoff reversibel zu binden, oder für eine Variante tierischen Ursprungs oder für einen Teil dieses proteinartigen Bestandteils, wobei besagte Variante oder besagter Teil in der Lage sind, den Sauerstoff reversibel zu binden, und eventuell eine codierende Sequenz für ein Selektionsmittel;
   ii) Selektion der Zellen mit codierender Nukleinsäure für den proteinartigen Bestandteil des Hämproteins;
   iii) eventuell Propagation transformierter Zellen, entweder in Kulturen oder durch Regeneration ganzer transgenischer oder chimärer Pflanzen;
   iv) Gewinnung und eventuell Aufbereitung eines Hämproteins, bestehend aus einem Komplex, der durch das Protein oder die durch oben genannte Nukleinsäure codierende Proteine gebildet wird und mindestens einem porphyrischen Eisenkern oder einer Vielfalt solcher Komplexe.
   v)

2. Verfahren nach Anspruch 1, **dadurch gekennzeichnet, dass** das Hämprotein aus einer einzigen Polypeptidkette besteht, z. B. dem Myoglobin.

3. Verfahren nach Anspruch 1, **dadurch gekennzeichnet, dass** das Hämprotein ein Heterooligomer ist, wobei die Nukleinsäure(n) die codierenden Sequenzen für jede einzelne Proteineinheit enthält (enthalten).

4. Verfahren nach Anspruch 3, **dadurch gekennzeichnet, dass** das Hämprotein menschliches Hämoglobin oder ein Derivat desselben ist, wobei die Nukleinsäure(n) die codierenden Sequenzen für das α- und β-Globin oder für Varianten des α- und β-Globins enthält (enthalten) und sich die Varianten von der natürlichen Sequenz durch eine oder mehrere Substitutionen, Deletionen oder Insertionen von Aminosäuren unterscheiden.

5. Verfahren nach Anspruch 4, **dadurch gekennzeichnet, dass** die codierenden Sequenzen für die einzelnen Proteineinheiten, α- und β-Globine, in demselben Nukleinsäuremolekül enthalten sind.

6. Verfahren nach Anspruch 4, **dadurch gekennzeichnet, dass** die codierenden Sequenzen für die einzelnen Proteineinheiten, α- und β-Globine, in verschiedenen Nukleinsäuremolekülen enthalten sind.

7. Verfahren nach einem der Ansprüche 1 bis 6, **dadurch gekennzeichnet, dass** die Einführung des oder der Nukleinsäuremoleküle durch Transformation des Kerngenoms der Pflanzenzelle erfolgt.

8. Verfahren nach Anspruch 7, **dadurch gekennzeichnet, dass** die codierende Sequenz für den proteinartigen Bestandteil eine oder mehrere codierende Sequenzen für chloroplastische oder mitochondriale Adresssignale enthält.

9. Verfahren nach Anspruch 7, **dadurch gekennzeichnet, dass** die codierende Sequenz für den proteinartigen Bestandteil eine oder mehrere codierende Sequenzen für ein N-terminal lokalisiertes Signalpeptid und eventuell ein Signal zum Zurückhalten des Proteins im Endoplasmatischen Retikulum oder ein vakuolaeres Adresssignal enthält.

10. Verfahren nach einem der Ansprüche 1 bis 6, **dadurch gekennzeichnet, dass** die Einführung der Nukleinsäure durch Transformation des mitochondrialen oder chloroplastischen Genoms geschieht.

11. Verfahren nach einem der Ansprüche 1 bis 9, **dadurch gekennzeichnet, dass** die Nukleinsäure, neben der oder den codierenden Sequenzen regulierende Transkriptionssequenzen enthält, die von den Pflanzenzellen erkannt werden.

12. Verfahren nach einem der Ansprüche 4 bis 11, **dadurch gekennzeichnet, dass** die codierende(n) Sequenz(en) für mindestens aus aktiven Teile des α-Globins und des β-Globins zusammengesetztes Hybridmolekül codiert (codieren).

**13.** Verfahren nach einem der Ansprüche 1 bis 12, **dadurch gekennzeichnet, dass** zwischen der Propagationsphase und der Gewinnungsphase eine Phase zur Detektion der Hämproteine liegt, und zwar insbesondere der Hämproteine, deren porphyrischer Kern aus Eisen-Protoporphyrin-IX gebildet wird.

**14.** Verfahren nach Anspruch 13, **dadurch gekennzeichnet, dass** während der Propagationsphase der Pflanzenzellen den zum Zellenwachstum angelegten Kulturen Eisen-Protoporphyrin-IX hinzugefügt wird.

**15.** Hämprotein mit der Fähigkeit, Sauerstoff reversibel zu binden, **dadurch gekennzeichnet, dass** es mindestens einen porphyrischen Eisenkern pflanzlichen Ursprungs und einen proteinartigen Bestandteil mit mindestens einer Polypeptidkette tierischen Ursprungs enthält.

**16.** Protein nach Anspruch 15, **dadurch gekennzeichnet, dass** es sich bei dem porphyrischen Eisenkern um Eisen-Protoporphyrin-IX handelt oder um ein Protoporphyrin, das sich vom Protoporphyrin IX durch die Beschaffenheit der von den β-Atomen der Pyrrole-Zyklen getragenen Seitenketten unterscheidet.

**17.** Protein nach Anspruch 16, **dadurch gekennzeichnet, dass** der proteinartige Bestandteil aus mindestens einer Polypeptidkette aus α- und/oder β-Globin oder entsprechenden Varianten besteht, die eine oder mehrere Substitutionen, Deletionen oder Insertionen von Aminosäuren enthalten, wobei das Hämprotein in der Lage ist, den Sauerstoff reversibel zu binden.

**18.** Protein nach Anspruch 17, **dadurch gekennzeichnet, dass** die α- oder β-Globin-Kette oder entsprechende Varianten außerdem ein chloroplastisches Adresssignal, ein mitochondriales Adresssignal oder ein N-terminal lokalisiertes Signalpeptid, eventuell in Verbindung mit einem Signal zum Zurückhalten des Proteins im Endoplasmatischen Retikulum, oder ein vakuoläres Adresssignal aufweist.

**19.** Protein nach Anspruch 17, **dadurch gekennzeichnet, dass** jede Polypeptidkette frei von Methionin $NH_2$-terminal ist.

**20.** Protein nach einem der Ansprüche 17 bis 19, **dadurch gekennzeichnet, dass** der proteinartige Bestandteil mindestens vier Polypeptidketten aus α- und/oder β-Globin oder entsprechende Varianten enthält, wobei jede Polypeptidkette an einen Eisen-Protoporphyrinkern gebunden ist.

**21.** Protein nach Anspruch 20, **dadurch gekennzeichnet, dass** es 2 α-Globin-Ketten und 2 β-Globin-Ketten oder entsprechende Varianten enthält.

**22.** Protein nach einem der Ansprüche 15 bis 21, **dadurch gekennzeichnet, dass** es den Sauerstoff mit einer Affinität von 7 bis 40 mm Hg, vorzugsweise von 15 bis 20 mm Hg, bindet.

**23.** Nukleinsäure, bestehend aus:

i) Einer oder mehreren Sequenzen, die einen proteinartigen Bestandteil eines Hämproteins tierischen Ursprungs codieren, wobei besagtes Protein in der Lage ist, den Sauerstoff reversibel zu binden und
ii) regulierenden, von der Pflanzenzelle erkannten Transkriptionssequenzen mit Promotor und regulierenden Terminationssequenzen und
iii) einer oder mehreren Sequenzen, die ein Adresssignal pflanzlichen Ursprungs codieren.
iv)

**24.** Nukleinsäure nach Anspruch 23, **dadurch gekennzeichnet, dass** die regulierenden Sequenzen einen oder mehrere Promotoren pflanzlichen Ursprungs enthalten.

**25.** Nukleinsäure nach Anspruch 23 oder 24, **dadurch gekennzeichnet, dass** die das Adresssignal codierenden Sequenzen für ein mitochondriales oder chloroplastisches Adresspeptid, das so genannte "Transit"-Peptid, codieren.

**26.** Nukleinsäure nach Anspruch 23 oder 24, **dadurch gekennzeichnet, dass** die das Adresssignal codierenden Sequenzen ein N-terminal lokalisiertes Signalpeptid pflanzlichen Ursprungs codieren, eventuell in Verbindung mit einer Sequenz, die ein endoplasmatisches Retentionssignal oder ein vakuolaeres Adresssignal codieren.

27. Nukleinsäure nach einem der Ansprüche 23 bis 26, **dadurch gekennzeichnet, dass** die codierende Sequenz menschliches α- oder β-Globin oder entsprechende Varianten codiert, wobei sie sich von der natürlichen Sequenz durch eine oder mehrere Substitutionen, Deletionen oder Insertionen von Aminosäuren unterscheiden, oder dass sie einen Teil des menschlichen α- oder β-Globins codiert.

28. Nukleinsäure nach einem der Ansprüche 23 bis 27 mit, unter anderem, einem oder mehreren Introns vorzugsweise pflanzlichen Ursprungs.

29. Nukleinsäure nach einem der Ansprüche 23 bis 28, **dadurch gekennzeichnet, dass** es sich bei der codierenden Sequenz für den proteinartigen Bestandteil um eine cDNA handelt.

30. Vektor mit einem oder mehreren Nukleinsäuremolekülen nach einem der Ansprüche 23 bis 29.

31. Pflanzenzellen, die durch die Nukleinsäure nach einem der Ansprüche 23 und 29 stabil transformiert wurden.

32. Pflanzenzellen, die in der Lage sind, ein oder mehrere Hämproteine nach einem der Ansprüche 15 bis 22 zu erzeugen.

33. Pflanzenzellen nach Anspruch 32, **dadurch gekennzeichnet, dass** sie eine Nukleinsäure enthalten, die aus einer oder mehreren codierenden Sequenzen für einen proteinartigen Bestandteil des besagten Hämproteins in Verbindung mit einer oder mehreren regulierenden, von der Zelle erkannten Transkriptionssequenzen besteht.

34. Pflanzenzellen nach einem der Ansprüche 31 bis 33, **dadurch gekennzeichnet, dass** es sich um eine Pflanzenzellkultur z. B. in flüssigem oder immobilem Milieu oder um eine Wurzelkultur handelt.

35. Pflanzenzellen nach einem der Ansprüche 31 bis 33, **dadurch gekennzeichnet, dass** es sich um Zellen einer ganzen transformierten Pflanze handelt.

36. Chimäre oder transgenische Pflanze, die in der Lage ist, ein oder mehrere Hämproteine wie z. B. Hämoglobin oder ein entsprechendes Derivat zu erzeugen, **dadurch gekennzeichnet, dass** sie Zellen gemäß einem der Ansprüche 31 bis 33 enthält.

37. Samen einer transgenischen Pflanze gemäß Anspruch 36.

38. Pharmazeutisches Erzeugnis mit einem oder mehreren Hämproteinen gemäß einem der Ansprüche 15 bis 22 in Verbindung mit einem aus physiologischer Sicht akzeptablen Arzneiträger.

39. Hämproteine nach einem der Ansprüche 15 bis 22 für eine Verwendung als Medikament.

40. Hämprotein nach Anspruch 39 für eine Verwendung im Rahmen einer Konditionstherapie zur Verbesserung des Sauerstofftransports im Blut.

41. Verwendung eines Hämproteins nach einem der Ansprüche 15 bis 22 zur Herstellung eines Medikamentes für die Konditionsbehandlung die eine Verbesserung des Sauerstofftransports im Blut erfordert.

42. Verwendung eines Hämproteins nach einem der Ansprüche 15 bis 22 in einem kosmetischen oder industriellen Produkt oder als chemisches Reagenz.

**Claims**

1. Haem protein production method that includes the following stages:

   i) The introduction, into vegetable cells, of one or several molecule(s) of nucleic acid each one of which includes at least one sequence coding for a proteinaceous component of a haem protein of animal origin capable of reversibly fixing oxygen, or for a variant of animal origin or part of this proteinaceous component, the aforementioned variant or part being capable of reversibly fixing oxygen, and possibly a sequence coding for a selection agent;

ii) The selection of cells containing the nucleic acid coding for the haem protein's proteinaceous component;

iii) Possibly, the propagation of the transformed cells, either in culture, or by the regeneration of entire transgenic or chimeric plants;

iv) The recovery, and possibly the purification, of a haem protein which includes a complex made up of the protein or proteins coded by the aforesaid nucleic acid and at least one iron porphyritic kernel, or a plurality of these complexes.

2. Method according to claim 1, **characterised in that** the haem protein has a single polypeptide chain, for example the myoglobin.

3. Method according to claim 1, **characterised in that** the haem protein is a heterooligomer, wherein the nucleic acid (s) include sequences coding for each of the different protein units.

4. Method according to claim 3, **characterised in that** the haem protein is human haemoglobin, or a derivative of the latter, wherein the nucleic acid(s) include sequences coding for α- and β-globin, or for variants of α- and β-globin, the variants are differentiated from the natural sequence by one or several substitution(s), deletion(s) or insertion(s) of amino acids.

5. Method according to claim 4, **characterised in that** the sequences coding for the different protein units, α- and β-globin, are included within the same molecule of nucleic acid.

6. Method according to claim 4, **characterised in that** the sequences coding for the different protein units, α- and β-globin, are included within distinct molecules of nucleic acid.

7. Method according to any one of the claims 1 to 6, **characterised in that** the introduction of the molecule(s) of nucleic acid is carried out by the transformation of the vegetable cell's nuclear genome.

8. Method according to claim 7, **characterised in that** the sequence coding for the protein component includes one or several sequences coding for the chloroplastic addressing signals, or for mitochondrial addressing signals.

9. Method according to claim 7, **characterised in that** the sequence coding for the protein component includes one or several sequences coding for an N-terminal signal peptide and possibly a signal responsible for the protein retention in the endoplasmatic reticulum, or a vescular addressing signal.

10. Method according to any one of the claims 1 to 6, **characterised in that** the introduction of the nucleic acid is carried out by the transformation of the mitochondrial or chloroplastic genome.

11. Method according to any one of the claims 1 to 9, **characterised in that** the nucleic acid includes, in addition to the coding sequence(s), regulatory transcription sequences which are recognised by vegetable cells.

12. Method according to any one of the claims 4 to 11, **characterised in that** the coding sequence(s) code(s) for a hybrid molecule made up of at least the active parts of the aglobin and β-globin.

13. Method according to any one of the claims 1 to 12, **characterised in that** it includes, between the propagation stage and the recovery stage, a haem protein detection stage, which in particular detects haem proteins whose porphyritic kernel is made up of iron IX protoporphyrin.

14. Method according to claim 13, **characterised in that**, during the vegetable cell propagation stage, iron IX protoporphyrin is added to the substrate of the culture used for cell growth.

15. Haem protein which has the ability to reversibly fix oxygen, **characterised in that** it includes at least one iron porphyritic kernel, of vegetable origin, and one protein component which includes at least one polypeptide chain of animal origin.

16. A protein according to claim 15, **characterised in that** the iron porphyritic kernel is the iron IX protoporphyrin, or a protoporphyrin which distinguishes itself from the IX protoporphyrin by the nature of the lateral chains carried by the pyrrole cycles' β atoms.

17. A protein according to claim 16, **characterised in that** the protein component includes at least one polypeptide chain α- and/or β-giobin, or variants of these, which include one or several substitution(s), deletion(s) or insertion (s) of amino acids, the haem protein being capable of reversibly fixing oxygen.

18. A protein according to claim 17, **characterised in that** the α- or β-globin chain, or variants of these, include in addition a chloroplastic addressing signal, a mitochondrial addressing signal, or an N-terminal peptide signal possibly in combination with a signal responsible for the protein retention in the endoplasmatic reticulum, or a vescular addressing signal.

19. A protein according to claim 17, **characterised in that** each polypeptide chain is devoid of methionine $NH_2$-terminal.

20. A protein according to any one of the claims 17 to 19, **characterised in that** the protein component includes at least four polypeptide chains of α- and/or β-globin, or variants of these, each polypeptide chain being linked to an iron protoporphyrin kernel.

21. A protein according to claim 20, **characterised in that** it includes 2 α-globin chains and 2 β-globin chains, or variants of these.

22. A protein according to any one of the claims 15 to 21, **characterised in that** it fixes oxygen with an affinity of between 7 and 40 mm Hg inclusive, preferably 15 to 20 mm Hg.

23. Nucleic acid that includes:

   i) One or several sequence(s) which code(s) a protein component of an animal haem protein, the aforementioned protein has the ability to reversibly fix oxygen, and
   ii) Regulatory transcription sequences which are recognised by a vegetable cell, which include a promoter and regulatory termination sequences, and
   iii) One or several sequence(s) which code(s) an addressing signal of vegetable origin.

24. Nucleic acid according to claim 23, **characterised in that** the regulatory sequences include one or several promoter (s) of vegetable origin.

25. Nucleic acid according to claim 23 or 24, **characterised in that** the sequences which code the addressing signal code for a mitochondrial or chloroplastic addressing peptide known as a "transit" peptide.

26. Nucleic acid according to claim 23 or 24, **characterised in that** the sequences which code the addressing signal code an N-terminal signal peptide of vegetable origin, possibly in combination with a sequence which codes an endoplasmatic retention signal or a vescular addressing signal.

27. Nucleic acid according to any one of the claims 23 to 26, **characterised in that** the coding sequence codes human α- or β-globin, or a variant of these which is differentiated from the natural sequence by one or several substitution (s), deletion(s) or replacement(s) of amino acids, or a part of the human α- and/or β-globin.

28. Nucleic acid according to any one of the claims 23 to 27 which includes in addition one or several intron(s) preferably of vegetable origin.

29. Nucleic acid according to any one of the claims 23 to 28 **characterised in that** the sequence coding for the protein component is a cDNA.

30. A vector that includes one or several molecules of nucleic acid according to any one of the claims 23 to 29.

31. Vegetable cells transformed in a stable fashion by nucleic acid according to either one of the claims 23 and 29.

32. Vegetable cells capable of producing one or several haem protein(s) according to any one of the claims 15 to 22.

**33.** Vegetable cells according to claim 32, **characterised in that** they include a nucleic acid which includes one or several sequence(s) coding for a protein component of the aforementioned haem protein in association with one or several regulatory transcription sequence(s) recognised by the cell.

**34.** Vegetable cells according to any one of the claims 31 to 33, **characterised in that** it involves a culture of vegetable cells, for example in a liquid or immobilised substrate, or a root culture.

**35.** Vegetable cells according to any one of the claims 31 to 33, **characterised in that** it involves cells that are part of a whole transformed plant.

**36.** Chimeric or transgenic plant capable of producing one or several haem protein (s), for example the haemoglobin or a derivative of the latter, **characterised in that** it includes cells according to any one of the claims 31 to 33.

**37.** Transgenic plant seeds according to claim 36.

**38.** A pharmaceutical product which includes one or several haem protein(s) according to any one of the claims 15 to 22 in association with an physiologically acceptable excipient.

**39.** Haem proteins according to any one of the claims 15 to 22 for use as medication.

**40.** Haem protein according to claim 39 for use in therapy of conditions that necessitate an improvement in the transport of oxygen in the blood.

**41.** Use of a haem protein according to any one of the claims 15 to 22 for the preparation of medication for the treatment of conditions that necessitate an improvement in the transport of oxygen in the blood.

**42.** Use of a haem protein according to any one of the claims 15 to 22 in a cosmetic or industrial product or as a chemical reagent.

## Structure de l'hème

FIGURE 1

## Séquence alpha globine:

```
  1   GTG CTG TCT CCT GCC GAC AAG ACC AAC GTC AAG GCC GCC TGG GGC  45
      Val Leu Ser Pro Ala Asp Lys Thr Asn Val  Lys Ala Ala Trp Gly

 46   AAG GTT GGC GCG CAC GCT GGC GAG TAT GGT GCG GAG GCC CTG GAG  90
      Lys Val Gly Ala His Ala Gly Glu Tyr Gly Ala Glu Ala Leu Glu

 91   AGG ATG TTC CTG TCC TTC CCC ACC ACC AAG ACC TAC TTC CCG CAC 135
      Arg Met Phe Leu Ser Phe Pro Thr Thr Lys Thr Tyr Phe Pro His

136   TTC GAC CTG AGC CAC GGC TCT GCC CAG GTT AAG GGC CAC GGC AAG 180
      Phe Asp Leu Ser His Gly Ser Ala Gln Val Lys Gly His Gly Lys

181   AAG GTG GCC GAC GCG CTG ACC AAC GCC GTG GCG CAC GTG GAC GAC 225
      Lys Val Ala Asp Ala Leu Thr Asn Ala Val Ala His Val Asp Asp

226   ATG CCC AAC GCG CTG TCC GCC CTG AGC GAC CTG CAC GCG CAC AAG 270
      Met Pro Asn Ala Leu Ser Ala Leu Ser Asp Leu His Ala His Lys

271   CTT CGG GTG GAC CCG GTC AAC TTC AAG CTC CTA AGC CAC TGC CTG 315
      Leu Arg Val Asp Pro Val Asn Phe Lys Leu Leu Ser His Cys Leu

316   CTG GTG ACC CTG GCC GCC CAC CTC CCC GCC GAG TTC ACC CCT GCG 360
      Leu Val Thr Leu Ala Ala His Leu Pro Ala Glu Phe Thr Pro Ala

361   GTG CAC GCC TCC CTG GAC AAG TTC CTG GCT TCT GTG AGC ACC GTG 405
      Val His Ala Ser Leu Asp Lys Phe Leu Ala Ser Val Ser Thr Val

406   CTG ACC TCC AAA TAC CGT
      Leu Thr Ser Lys Tyr Arg
```

FIGURE 2

## Séquence beta globine:

```
1    GTG CAC CTG ACT CCT GAG GAG AAG TCT GCC GTT ACT GCC CTG TGG  45
     Val His Leu Thr Pro Glu Glu Lys Ser Ala Val Thr Ala Leu Trp

46   GGC AAG GTG AAC GTG GAT GAA GTT GGT GGT GAG GCC CTG GGC AGG  90
     Gly Lys Val Asn Val Asp Glu Val Gly Gly Glu Ala Leu Gly Arg

91   CTG CTG GTT GTC TAC CCT TGG ACC CAG AGG TTC TTT GAG TCC TTT  135
     Leu Leu Val Val Tyr Pro Trp Thr Gln Arg Phe Phe Glu Ser Phe

136  GGG GAT CTG TCC ACT CCT GAT GCT GTT ATG GGC AAC CCT AAG GTG  180
     Gly Asp Leu Ser Thr Pro Asp Ala Val Met Gly Asn Pro Lys Val

181  AAG GCT CAT GGC AAG AAA GTG CTC GGT GCC TTT AGT GAT GGC CTG  225
     Lys Ala His Gly Lys Lys Val Leu Gly Ala Phe Ser Asp Gly Leu

226  GCT CAC CTG GAC AAC CTC AAG GGC ACC TTT GCC ACA CTG AGT GAG  270
     Ala His Leu Asp Asn Leu Lys Gly Thr Phe Ala Thr Leu Ser Glu

271  CTG CAC TGT GAC AAG CTG CAC GTG GAT CCT GAG AAC TTC AGG CTC  315
     Leu His Cys Asp Lys Leu His Val Asp Pro Glu Asn Phe Arg Leu

316  CTG GGC AAC GTG CTG GTC TGT GTG CTG GCC CAT CAC TTT GGC AAA  360
     Leu Gly Asn Val Leu Val Cys Val Leu Ala His His Phe Gly Lys

361  GAA TTC ACC CCA CCA GTG CAG GCT GCC TAT CAG AAA GTG GTG GCT  405
     Glu Phe Thr Pro Pro Val Gln Ala Ala Tyr Gln Lys Val Val Ala

406  GGT GTG GCT AAT GCC CTA GCC CAC AAG TAT CAC
     Gly Val Ala Asn Ala Leu Ala His Lys Tyr His
```

FIGURE 3

EP 0 839 204 B1

FIGURE 4

EP 0 839 204 B1

FIGURE 5

EP 0 839 204 B1

FIGURE 6

FIGURE 7

FIGURE 8

FIGURE 9

cinétiques de recombinaison du CO

0.1 atm CO

FE-rHb

50 % dissociation

32%

11%

FIGURE 10

FIGURE 11